# EUROPEAN PATENT SPECIFICATION

(11) **EP 3 221 352 B1**
(45) Date of publication and mention of the grant of the patent: **09.05.2018**
(21) Application number: 16727798.7
(22) Date of filing: 01.06.2016
(51) Int. Cl.: C07K 16/18, C07K 16/30, G01N 33/574

(54) **MONOCLONAL ANTIBODIES BINDING MCM5**
MONOKLONALE ANTIKÖRPER GEGEN MCM5
ANTICORPS MONOCLONAUX DIRIGÉS CONTRE MCM5

(30) Priority: 08.06.2015 GB 201509907
(43) Date of publication of application: 27.09.2017
(73) Proprietor: Arquer Diagnostics Limited, Sunderland, Tyne and Wear SR5 2TA (GB)
(72) Inventor: LASKEY, Ronald Alfred, Cambridge Cambridgeshire CB2 3EJ (GB); STOEBER, Kai, Ely Cambridgeshire CB6 3HB (GB)
(74) Representative: J A Kemp
(86) International application number: PCT/GB2016/051609
(87) International publication number: WO 2016/198834

(56) References cited:
- WO-A1-99/21014
- WO-A1-2008/132453
- WO-A2-2005/026211
- STOEBER KAI ET AL: "Diagnosis of genito-urinary tract cancer by detection of minichromosome maintenance 5 protein in urine sediments", JOURNAL OF THE NATIONAL CANCER INSTITUTE, OXFORD UNIVERSITY PRESS, GB, vol. 94, no. 14, 17 July 2002 (2002-07-17) , pages 1071-1079, XP009122537, ISSN: 0027-8874, DOI: 10.1093/JNCI/94.14.1071
- T J DUDDERIDGE ET AL: "Diagnosis of prostate cancer by detection of minichromosome maintenance 5 protein in urine sediments", BRITISH JOURNAL OF CANCER, vol. 103, no. 5, 24 August 2010 (2010-08-24), pages 701-707, XP055294840, GB ISSN: 0007-0920, DOI: 10.1038/sj.bjc.6605785
- MAXIMILIAN BURGER: "MCM2 and MCM5 as Prognostic Markers in Colon Cancer: A Worthwhile Approach", DIGESTIVE DISEASES AND SCIENCES, KLUWER ACADEMIC PUBLISHERS-PLENUM PUBLISHERS, NE, vol. 54, no. 2, 21 August 2008 (2008-08-21), pages 197-198, XP019673457, ISSN: 1573-2568

## Description

### Field of the invention

The invention relates to monoclonal antibodies which bind to Mcm proteins, to compositions or kits comprising the monoclonal antibodies or to methods of using the antibodies.

### Background of the invention

Urological cancers (occasionally referred to as 'urinary system cancers') are a major and increasing epidemiological problem. Two of the most economically important urological cancers are bladder cancer and prostate cancer.

Prostate cancer is the second most common cancer in men after non-melanoma skin cancer, with over 35,000 new cases diagnosed each year in the UK; about 10,200 deaths annually are caused by prostate cancer. There are around 300,000 new cases in Europe, 190,000 in the US and 670,000 worldwide annually. Cancer Research UK report that a quarter of all new cases of cancer diagnosed in men in the UK are prostate cancers and 60% of new diagnoses are in men aged over 70 years. The most common form of the disease is adenocarcinoma. The 5-year survival rate is almost 80% in the UK. There is no known environmental cause but those with close relatives with prostate or breast cancer are more at risk of developing the disease. West African and Afro-Caribbean males have an increased risk of prostate cancer.

The symptoms of prostate cancer are similar to those caused by benign enlargement of the prostate gland, and include urgency to urinate, difficulty or pain in passing urine and rarely, blood in the urine or semen. However, in many men the disease remains symptomless until painful metastases form, predominantly in the bones.

Treatment depends on the stage and grade of the tumour and the patient's general health and age. Options include active surveillance, partial or radical prostatectomy, orchidectomy, hormone treatment, and radiotherapy such as brachytherapy. Orchidectomy and hormone treatment reduce or eliminate the production of testosterone, which is essential for tumour growth.

The definite diagnosis of prostate cancer requires a multi-faceted approach. The current gold standard diagnostic test for prostate cancer is the histological examination of biopsy material. The decision to biopsy is based on age-related serum (Prostate Specific Antigen) PSA level and/or an abnormal digital rectal examination (DRE). DRE, in which the gland is palpated trans-rectally to examine for abnormal morphology is also non-specific. Tumours that are too small to alter the morphology of the gland will not be detected, and abnormal morphology or enlargement is also caused by non-malignant conditions. This is a problem in the art. Samples of the prostate gland are commonly taken using TRUS (trans-rectal ultra sound)-guided needle biopsy. A number of needle cores are taken, typically up to 12, in order to maximize the area of the gland sampled. The procedure is carried out in the outpatients department under local anaesthesia by a urologist with the aid of a nurse or healthcare assistant. This procedure suffers from drawbacks including being somewhat painful for the patient, and exposing the patient to a risk of sepsis and/or bleeding. The tissue cores are microscopically examined in a laboratory for the presence of malignant cells, which has the problem of being labour intensive and requiring highly trained cytologists, as well as being vulnerable to human error.

It can be appreciated that biopsies are invasive and costly. There is a need in the art for a more cost-effective, reliable and/or non-invasive tool for the diagnosis and/or surveillance of urological cancer such as prostate cancer. Known alternate and/or less invasive diagnostic procedures for prostate cancer involve the analysis of specific biological markers ('biomarkers').

An example of a nucleic acid biomarker of prostate cancer is the PCA3 (prostate cancer gene 3) test. This urinary assay identifies non-coding mRNA from the PCA3 gene that is overexpressed in prostate cancer (Hessels & Schalken, The use of PCA3 in the diagnosis of prostate cancer. Nat Rev Urol, 6, 255-61; 2009). The PCA3 test (Gen-Probe, Inc) relies on the analysis of a first-catch urine specimen produced after a defined form of prostate massage used to express prostatic secretions, which contain epithelial cells into the urethra. As a diagnostic for prostate cancer PCA3 has a ROC (receiver operating characteristic curve) value of 0.68 (Chun et al, Prostate Cancer Gene 3 (PCA3): development and internal validation of a novel biopsy nomogram. Eur Urol; 2009 vol 56 p659-668) which is similar to that for the PSA test discussed below. However, the PCA3 test is costly and not amenable to point-of-care use, which are problems with this prior art technique.

An example of a protein biomarker, which is frequently used to indicate the presence of prostate cancer is PSA (Prostate Specific Antigen). Symptomatic patients presenting in primary care are typically given a serum PSA test and a DRE. However, PSA is not specific for prostate cancer. PSA is a constitutively expressed tissue specific intracellular enzyme. A low concentration of PSA is present in the serum of men with healthy prostate glands. A raised level of PSA in serum occurs due to leakage from the prostate gland and is an indication of the relative size of the gland. Raised PSA can occur in non-malignant conditions such as benign prostatic hyperplasia and prostatitis and also in prostate cancer. As men grow older, the volume of the gland increases resulting in rising PSA levels in the absence of malignant disease. In a recent study it was found that 60 - 70% of 'positive' PSA tests (serum level of PSA greater than 4 ng/mL) were not associated with cancer (Kilpeläinen et al., False-positive screening results in the Finnish prostate cancer screening trial, British Journal of Cancer. 102, 469-474; 2010). The high rate of false positive results leads to many unnecessary biopsy operations and renders the test inappropriate for population screening. In addition the PSA test fails to detect a significant number of cases of prostate cancer, particularly in younger men. The accuracy of the PSA test as measured in ROC (receiver operating characteristic) analysis is 0.678 (Thompson et al., Operating characteristics of a prostate-specific antigen in men with an initial PSA level of 3.0 ng/ml or lower. JAMA, 294, 66-70; 2005). In the UK, PSA tests are usually carried out in hospital laboratories although rapid point-of-care assays are available.

Bladder cancer is the fourth most common cancer in men and the ninth most common cancer in women and results in significant morbidity and mortality (Jemal et al. CA Cancer J Clin. 2007. 57:43-66.). Most patients with bladder cancer receive the diagnosis after they present with gross or microscopic haematuria or with other irritative voiding symptoms, such as frequency and dysuria. At initial diagnosis, approximately 70% of patients have bladder cancers that are confined to the epithelium or subepithelial connective tissue. These cancers can be managed with endoscopic resection and intravesical therapy. The recurrence rate for these tumours ranges from 50% to 70% and 10% to 15% of cases progress to muscle invasion over a 5-year period (Shariat et al., 2008. Rev Urol. 10:120-135). Recurrence may be seen locally and more rarely in the upper urinary tract even after several years, necessitating lifelong surveillance. The remaining 30% of patients have muscle-invasive cancer at initial diagnosis. Of this population, 50% have distant metastasis within 2 years, and 60% die within 5 years despite treatment.

The definite diagnosis of bladder cancer requires a combination of procedures. Presently there are no methods to identify accurately and easily the presence of early bladder cancer. Screening for bladder cancer in patients who present to the urology clinic with appropriate symptoms is currently done with urinalysis, cystoscopy and a scanning procedure such as abdominal ultrasound, intravenous urogram, computed tomography or magnetic resonance imaging. Urine cytology, in which cells from urine samples are examined microscopically, is used occasionally. Cystoscopy, the mainstay for the detection of bladder cancer, is a relatively short, minimally traumatic procedure performed with local urethral anaesthesia, which identifies nearly all papillary and sessile lesions. Nevertheless, it is still invasive and a cause of discomfort and distress to the patient. In addition, cystoscopy may be inconclusive at times because of the grossly abnormal appearance of the bladder mucosa, especially in patients with an indwelling catheter or active inflammation, and it is unable to detect cancers within the ureters. Although considered the gold standard for diagnosis of bladder cancer because it allows direct visualization and biopsy of the bladder urothelium, cystoscopy has an appreciable false-negative rate either from operator error or from small areas of "carcinoma in situ", which may be difficult to detect. (van der Poel & Debruyne. Curr Opin Urol. 2001; 11:503-509; Herr. BJU Int. 1999; 84:1102-1103.)

In urine cytology for bladder cancer, exfoliated cells can be investigated for the presence of specific cell-surface antigens, nuclear morphology, gene expression or other biological markers. Urine cytology has a high sensitivity and specificity for the detection of high-grade bladder cancer, but it lacks the sensitivity to detect low grade tumours (Wiener et al. Acta Cytol. 1993; 37:163-169). The accuracy of urine cytology in predicting bladder cancer recurrence may vary widely, in part because there is an element of subjectivity in the interpretation of the results. Hence, cytology is not ideal for screening for and surveillance of bladder cancer.

Mcm5 is a biomarker for cancer (WO99021014). A raised level of Mcm proteins such as Mcm5 in urine sediment is associated with malignant changes in the prostate gland. Hence raised levels of these Mcm proteins could be used to detect prostate cancer. Using DELFIA® (Dissociation-Enhanced Lanthanide Fluorometric Immunoassay) and anti-Mcm5 monoclonal antibodies 4B4 and 12A7 in a double antibody assay, Dudderidge et al. (BJC, 103, 701 - 707; 2010) investigated the use of Mcm5 as a urinary biomarker for prostate cancer detection and concluded that it 'seems to be a simple, accurate and non-invasive method for identifying patients with prostate cancer'. Compared with the PSA test, which has a specificity of 30%, the specificity of Mcm5 was estimated at between 73% and 93%. Importantly, benign prostatic hyperplasia did not generate false positive results, which is a disadvantage of the PSA test.

However there is no disclosure of sequences of antibodies that bind to Mcm5 in a highly specific manner and, therefore, are suitable for designing a high affinity assay for Mcm5.

### Summary of the invention

The present invention provides antibodies which bind to biomarkers such as an Mcm protein, for example Mcm5, in a highly specific manner. These antibodies are useful in detecting levels of the biomarker. For example, these antibodies can be used in diagnostic tests for detecting urological cancers.

Currently, detection of urological cancers, particularly prostate or bladder cancer, is difficult and may require invasive techniques. Mcm5 is a biomarker for cancer. The present inventors have developed two antibodies that bind to Mcm5 but bind to different epitopes to one another (12A7 binds to SEQ ID NO: 1 and 4B4 binds to SEQ ID NO: 2). Such antibodies are advantageous as they can be used in assays to detect Mcm5 (or similar Mcm proteins containing these epitopes). In particular, these antibodies can be used in an assay which requires immobilisation of the Mcm5 antigen. For example, Mcm5 could be detected using a two-site immunometric assay. A first antibody that binds to Mcm5 could be bound to a plate. This plate could be exposed to a sample such as a urine sample. Any Mcm5 in the sample will be immobilised ("*captured*") by the first antibody. The plate may then be washed, leaving the immobilised Mcm5 bound to the first antibody, but removing any other contaminants. A second antibody that binds to Mcm5 can be used to detect the concentration of bound Mcm5. This second antibody should be conjugated to a detection agent such as Eu³⁺. The plate can be exposed to the second antibody and the second antibody will bind to the immobilised Mcm5. Any excess antibody can be removed by washing. The amount of second antibody remaining can be detected by fluorescence quantification which will measure the amount of Eu³⁺ present. The amount of second antibody remaining will be proportional to the concentration of Mcm5 in the sample.

However, in order for such an assay to be developed, two antibodies that bind to Mcm5 must be identified. Furthermore, these two antibodies must bind to different epitopes to one another, but also the two different epitopes to which the two antibodies bind must be spatially positioned such that both antibodies may bind to Mcm5 at the same time (without substantial steric hindrance). The present inventors have developed two such antibodies (named 12A7 and 4B4). These two antibodies bind to SEQ ID NO: 1 and SEQ ID NO: 2 respectively. SEQ ID NO: 1 and SEQ ID NO: 2 represent two different Mcm5 epitopes which are spatially arranged on Mcm5 to allow the two antibodies to bind to Mcm5 at the same time. This is demonstrated in Examples 2 and 3. The present inventors are the first to identify two Mcm5 epitopes which are arranged such that antibodies to each epitope may bind simultaneously. Furthermore, the inventors are the first to provide sequences of two antibodies that can bind independently to Mcm5.

Accordingly in a first aspect of the invention there is provided an antibody which:
(i) binds to an epitope having an amino acid sequence of SEQ ID NO: 1 or 2; or
(ii) comprises:
   a.
      12A7 CDRH1 which has a sequence of SEQ ID NO: 9;
      12A7 CDRH2 which has a sequence of SEQ ID NO: 11;
      12A7 CDRH3 which has a sequence of SEQ ID NO: 13;
      12A7 CDRL1 which has a sequence of SEQ ID NO: 3;
      12A7 CDRL2 which has a sequence of SEQ ID NO: 5; and
      12A7 CDRL3 which has a sequence of SEQ ID NO: 7; or
   b.
      4B4 CDRH1 which has a sequence of SEQ ID NO: 21;
      4B4 CDRH2 which has a sequence of SEQ ID NO: 23;
      4B4 CDRH3 which has a sequence of SEQ ID NO: 25;
      4B4 CDRL1 which has a sequence of SEQ ID NO: 15;
      4B4 CDRL2 which has a sequence of SEQ ID NO: 17; and
      4B4 CDRL3 which has a sequence of SEQ ID NO: 19.

In a second aspect of the invention there is provided a composition comprising the antibody of the invention.

In a third aspect of the invention there is provided a hybridoma cell line capable of producing the antibody of the invention.

In a fourth aspect of the invention there is provided a composition comprising a nucleic acid sequence encoding a polypeptide comprising the heavy chain of an antibody of the invention and a nucleic acid sequence encoding a polypeptide comprising the light chain of an antibody the invention.

In a fifth aspect of the invention there is provided a composition comprising a nucleic acid sequence at least 98%, 99% or 100% identical to 28 or 32 and a nucleic acid sequence at least 98%, 99%, or 100% identical to SEQ ID NO: 30 or 34.

In a sixth aspect of the invention there is provided a composition comprising a nucleic acid sequence of SEQ ID NO: 28 or 32 and a nucleic acid sequence of SEQ ID NO: 30 or 34.

In a seventh aspect of the invention there is provided a vector comprising a nucleic acid of the invention.

In an eighth aspect of the invention there is provided a host cell comprising a nucleic acid or vector of the invention.

In a ninth aspect of the invention there is provided a method of making an antibody comprising:
(i) growing a hybridoma cell line or host cell of the invention; and
(ii) isolating an antibody from the hybridoma cell line or host cell.

In a tenth aspect of the invention there is provided a kit comprising:
(i) a composition comprising a first antibody of the invention; and
(ii) a composition comprising a second antibody that binds to Mcm5.

In an eleventh aspect of the invention there is provided a kit comprising:
(i) a composition comprising a first antibody of the invention; and
(ii) a composition comprising a second antibody of the invention.

In a twelfth aspect of the invention there is provided a method of determining the concentration of Mcm5 in a sample comprising the following steps:
(i) providing an immobilised first antibody which is an antibody of the invention immobilised to a solid support;
(ii) exposing the sample to the immobilised first antibody such that Mcm5 in the sample may bind to the immobilised antibody to provide immobilised Mcm5;
(iii) providing a labelled second antibody which is an antibody the invention conjugated to a label;
(iv) exposing the immobilised Mcm5 to the labelled second antibody such that the labelled second antibody may bind to the Mcm5; and
(v) detecting the concentration of the labelled second antibody.

### Detailed description

### Antibody

The present invention provides an antibody. The term *'antibody'* can refer to naturally occurring forms or recombinant antibodies such as single-chain antibodies, chimeric antibodies or humanised antibodies. The terms *'antibody'* and *'antibodies'* may also be considered to encompass fragments of antibodies that can bind to a target protein, such as an Mcm protein like Mcm5. Such fragments may include Fab'₂, F'(ab)₂, Fv, single chain antibodies or diabodies. In a preferred embodiment antibodies of the invention are naturally occurring, full length antibodies (rather than fragments). In a preferred embodiment the antibody is a mouse antibody (*i.e.* originally derived from mouse spleen cells).

In general, antibodies are formed from two heavy chains and two lights chains. Each heavy chain is made up of a heavy chain constant region (CH) and a heavy chain variable region (VH). Similarly each light chain is made up of a light chain constant region (CL) and a light chain variable region (VL). The VH and VL regions comprise complementarity defining regions (CDRs). The CDRs are primarily responsible for specific binding to the target protein.

An antibody of the invention may be an antibody of any class, particularly IgG, IgM or IgA. In a preferred embodiment the antibody is an IgG antibody.

The term *"monoclonal antibody"* is intended to refer to an antibody that is obtained from a population of substantially similar antibodies, *i.e*. from a population of antibodies that are identical except for a minority of naturally occurring variants. A population of monoclonal antibodies will bind to the same epitope. Monoclonal antibodies can be produced by a variety of techniques well known to the person skilled in the art and including the methods disclosed in "Monoclonal Antibodies: A manual of techniques", H Zola (CRC Press, 1988) and in *"*Monoclonal Hybridoma Antibodies: Techniques and Application", SG Hurrell (CRC Press, 1982). The antibody of the invention is a monoclonal antibody.

### Antibody target

The antibody binds specifically to Mcm5. The antibody binds to at least one of SEQ ID NO: 1 or SEQ ID NO: 2. The antibody specifically binds to at least one of SEQ ID NO: 1 or SEQ ID NO: 2.

Mcm5 is a putative DNA replication licensing factor and a member of the MCM (minichromosome maintenance) protein family. MCM proteins bind to chromatin and are believed to exhibit helicase activity and thus be core components of DNA replication. Mcm5 is upregulated in the transition from the G0 to G1/S phase of the cell cycle and may actively participate in cell cycle regulation; Mcm5 can interact with at least two other members of the MCM family.

MCM proteins 2 - 7 comprise part of the pre-replication complexes which form on chromatin and which are essential prerequisites, or licensing factors, for subsequent DNA replication. The MCM protein complexes act as replicative helicases and thus are core components of the DNA replication machinery. MCMs are upregulated in the transition from the G0 to G1/S phase of the cell cycle and actively participate in cell cycle regulation. The MCM proteins form an annular structure around the chromatin.

The human Mcm5 gene maps to 22q13.1 and the mature Mcm5 protein consists of 734 amino acids (SEQ ID NO: 35; UNIPROT P33992: HUMAN DNA replication licensing factor MCM5). However, the Mcm5 gene may differ slightly in sequences between individuals. For this reason, the phrase *"binds to Mcm5*" refers to antibodies that bind to SEQ ID NO: 35, but also to antibodies that bind to a polypeptide having sequences at least 90%, 95%, 95%, 98%, 99% or 100% identical to SEQ ID NO: 35. In a preferred embodiment the antibody binds to a polypeptide 100% identical to SEQ ID NO: 35. Furthermore, an antibody of the invention will bind to an epitope (fragment) of Mcm5 (for example SEQ ID NO: 1 or SEQ ID NO: 2). Thus, the term *"antibody which binds to Mcm5*" refers to an antibody that binds to only a single epitope of Mcm5 such as SEQ ID NO: 1 or SEQ ID NO: 2.

For the purposes of the present invention the term '*binding affinity'* refers to the ability of an antibody to bind to its target. For the purposes of the present invention, the term *'specifically binds'* refers to an antibody that binds to a target such as Mcm5 with a binding affinity that is at least 2-fold, 10-fold, 50-fold or 100-fold greater than its binding affinity for binding to another non-target molecule. In an embodiment the non-target molecule is an Mcm protein, other than Mcm5, such as Mcm 2. Preferably, an antibody of the invention is capable of binding to an Mcm protein, optionally Mcm5, with a binding affinity that is at least 2-fold, 10-fold, 50-fold or 100-fold greater than its binding affinity for binding to another non-target molecule. Even more preferably, an antibody of the invention is capable of binding to SEQ ID NO: 1 or SEQ ID NO: 2 with a binding affinity that is at least 2-fold, 10-fold, 50-fold or 100-fold greater than its binding affinity for binding to another non-target molecule.

A preferred method for the evaluation of binding affinity for Mcm5 is by ELISA. Preferably, an antibody of the invention has an affinity for Mcm5 (measured as an EC50 or 50% maximum binding concentration, as described in Example 2) of 2500 ng/ml or lower, 1500 ng/ml or lower, 1000 ng/ml or lower, 600 ng/ml or lower, 50 ng/ml or lower, 30 ng/ml or lower, 20 ng/ml or lower, or 10 ng/ml or lower. The EC50 will typically be higher than 1 ng/ml and thus the EC50 may be between 1 ng/ml and any of the upper limits specified in the preceding sentence. Other standard assays to evaluate the binding ability of ligands such as antibodies towards targets are known in the art, including for example, Western blots, RIAs, and flow cytometry analysis. The binding kinetics (*e.g.* binding affinity) of the antibody also can be assessed by standard assays known in the art, such as by Surface Plasmon Resonance (SPR) (*e.g.* Biacore ™ system) analysis. The affinity constant (KD) for binding to Mcm5 is preferably in the range of 1-10 000 nM, 1-1000 nM, 1-500 nM, 1-100 nM, 1-50 nM or 1-10 nM. The association rate (ka) is preferably in the range of 0.4-3.4 x 10⁶ 1/M. The dissociation rate (kd) is preferably in the range of 1-10 x 10⁻³ 1/s. These values may typically be determined by SPR (Surface Plasmon Resonance).

### Complementary determining regions (CDRs)

An antibody of the invention comprises CDRs of antibodies 12A7 or 4B4, *i.e*.:
a. 12A7 CDRH1 which has a sequence of SEQ ID NO: 9;
b. 12A7 CDRH2 which has a sequence of SEQ ID NO: 11;
c. 12A7 CDRH3 which has a sequence of SEQ ID NO: 13;
d. 12A7 CDRL1 which has a sequence of SEQ ID NO: 3;
e. 12A7 CDRL2 which has a sequence of SEQ ID NO: 5; and
f. 12A7 CDRL3 which has a sequence of SEQ ID NO: 7; or
g. 4B4 CDRH1 which has a sequence of SEQ ID NO: 21;
h. 4B4 CDRH2 which has a sequence of SEQ ID NO: 23;
i. 4B4 CDRH3 which has a sequence of SEQ ID NO: 25;
j. 4B4 CDRL1 which has a sequence of SEQ ID NO: 15;
k. 4B4 CDRL2 which has a sequence of SEQ ID NO: 17; and
l. 4B4 CDRL3 which has a sequence of SEQ ID NO: 19.

Antibodies that have the same CDRs as the 4B4 and 12A7 antibodies may differ substantially from the sequences of 4B4 and 12A7 in other regions. Such antibodies may, for example, be antibody fragments.

The phrase *"sequence that differs from SEQ ID NO: 3 by a single amino acid substitution*" refers to the possibility of replacing one amino acid defined in SEQ ID NO: 3 by a different amino acid. Preferably such a replacement is a conservative amino acid substitution. The following eight groups each contain amino acids that are typically conservative substitutions for one another:
1) Alanine, Glycine;
2) Aspartic acid, Glutamic acid;
3) Asparagine, Glutamine;
4) Arginine, Lysine;
5) Isoleucine, Leucine, Methionine, Valine;
6) Phenylalanine, Tyrosine, Tryptophan;
7) Serine, Threonine; and
8) Cysteine, Methionine.

In a disclosure the antibody of the invention comprises at least one CDR from the heavy chain of 12A7 (12A7 CDRH1, 12A7 CDRH2 or 12A7 CDRH3) as well as at least one CDR from the light chain of 12A7 (12A7 CDRL1, 12A7 CDRL2 or 12A7 CDRL3). In a further disclosure the antibody of the invention comprises at least two CDRs from the heavy chain of 12A7 and at least two CDRs from the light chain of 12A7. In a disclosure the antibody of the invention comprises all three CDRs from the heavy chain of 12A7 and/or all three CDRs from the light chain of 12A7. In a disclosure the antibody of the invention comprises 12A7 CDRL1 and 12A7 CDRL2, 12A7 CDRL1 and 12A7 CDRL3, 12A7 CDRL1 and 12A7 CDRH1, 12A7 CDRL1 and 12A7 CDRH2, 12A7 CDRL1 and 12A7 CDRH3, 12A7 CDRL2 and 12A7 CDRL3, 12A7 CDRL2 and 12A7 CDRH1, 12A7 CDRL2 and 12A7 CDRH2, 12A7 CDRL2 and 12A7 CDRH3, 12A7 CDRL3 and 12A7 CDRH1, 12A7 CDRL3 and 12A7 CDRH2, 12A7 CDRL3 and 12A7 CDRH3, 12A7 CDRH1 and 12A7 CDRH2, 12A7 CDRH1 and 12A7 CDRH3, or 12A7 CDRH2 and 12A7 CDRH3.

In a disclosure the antibody of the invention comprises at least one CDR from the heavy chain of 4B4 (4B4 CDRH1, 4B4 CDRH2 or 4B4 CDRH3) as well as at least one CDR from the light chain of 4B4 (4B4 CDRL1, 4B4 CDRL2 or 4B4 CDRL3). In a further disclosure the antibody of the invention comprises at least two CDRs from the heavy chain of 4B4 and at least two CDRs from the light chain of 4B4. In a disclosure the antibody of the invention comprises all three CDRs from the heavy chain of 4B4 and/or all three CDRs from the light chain of 4B4. In a disclosure the antibody of the invention comprises 4B4 CDRL1 and 4B4 CDRL2, 4B4 CDRL1 and 4B4 CDRL3, 4B4 CDRL1 and 4B4 CDRH1, 4B4 CDRL1 and 4B4 CDRH2, 4B4 CDRL1 and 4B4 CDRH3, 4B4 CDRL2 and 4B4 CDRL3, 4B4 CDRL2 and 4B4 CDRH1, 4B4 CDRL2 and 4B4 CDRH2, 4B4 CDRL2 and 4B4 CDRH3, 4B4 CDRL3 and 4B4 CDRH1, 4B4 CDRL3 and 4B4 CDRH2, 4B4 CDRL3 and 4B4 CDRH3, 4B4 CDRH1 and 4B4 CDRH2, 4B4 CDRH1 and 4B4 CDRH3, or 4B4 CDRH2 and 4B4 CDRH3.

In a disclosure the antibody of the invention comprises at least one CDR having a sequence identical to that described in any one of SEQ ID NO: 3 (12A7 CDRL1), SEQ ID NO :5 (12A7 CDRL2), SEQ ID NO: 7 (12A7 CDRL3), SEQ ID NO: 9 (12A7 CDRH1), SEQ ID NO: 11 (12A7 CDRH2), SEQ ID NO: 13 (12A7 CDR H3), SEQ ID NO: 15 (4B4 CDRL1), SEQ ID NO: 17 (4B4 CDRL2), SEQ ID NO: 19 (4B4 CDRL3), SEQ ID NO: 21 (4B4 CDRH1), SEQ ID NO: 23 (4B4 CDRH2) or SEQ ID NO :25 (4B4 CDRH3). Where an antibody of the invention comprises 12A7 CDRL2, the 12A7 CDRL2 has the sequence described in SEQ ID NO: 5. Where an antibody of the invention comprises 12A7 CDRL1, the 12A7 CDRL1 has the sequence described in SEQ ID NO: 3. Where an antibody of the invention comprises 12A7 CDRL3, the 12A7 CDRL3 has the sequence described in SEQ ID NO: 7. Where an antibody of the invention comprises 12A7 CDRH1, the 12A7 CDRH1 has the sequence described in SEQ ID NO: 9. Where an antibody of the invention comprises 12A7 CDRH2, the 12A7 CDRH2 has the sequence described in SEQ ID NO: 11. Where an antibody of the invention comprises 12A7 CDRH3, the 12A7 CDRH3 has the sequence described in SEQ ID NO: 13. Where an antibody of the invention comprises 4B4 CDRL1, the 4B4 CDRL1 has the sequence described in SEQ ID NO: 15. Where an antibody of the invention comprises 4B4 CDRL2, the 4B4 CDRL2 has the sequence described in SEQ ID NO: 17. Where an antibody of the invention comprises 4B4 CDRL3, the 4B4 CDRL3 has the sequence described in SEQ ID NO: 19. Where an antibody of the invention comprises 4B4 CDRH1, the 4B4 CDRH1 has the sequence described in SEQ ID NO: 21. Where an antibody of the invention comprises 4B4 CDRH2, the 4B4 CDRH2 has the sequence described in SEQ ID NO: 23. Where an antibody of the invention comprises 4B4 CDRH3, the 4B4 CDRH3 has the sequence described in SEQ ID NO: 25.

Preferably an antibody comprising at least one of the CDRs of 12A7 or 4B4 binds (optionally specifically binds) to Mcm5. Even more preferably an antibody comprising at least one of the CDRs of 12A7 or 4B4 binds (optionally specifically binds) to SEQ ID NO: 1 or SEQ ID NO: 2.

### Heavy and light chain variable region sequences

The antibody of the invention preferably comprises a heavy chain variable region having a sequence at least 85%, 90%, 95%, 98%, 99% or 100% identical to SEQ ID NO: 29 or 33 and/or a light chain variable region sequence having a sequence at least 85%, 90%, 95%, 98%, 99% or 100% identical to SEQ ID NO: 27 or 31. Such an antibody is referred to herein as a *"variant antibody of the invention".*

In an embodiment the antibody comprises a heavy chain variable region having a sequence at least 95% identical to SEQ ID NO: 29. In a further embodiment the antibody comprises a heavy chain variable region having a sequence at least 98% identical to SEQ ID NO: 29. In one embodiment the antibody comprises a light chain variable region having a sequence at least 95% identical to SEQ ID NO: 27. In a further embodiment the antibody comprises a light chain variable region having a sequence at least 98% identical to SEQ ID NO: 27. In a further embodiment the antibody comprises a light chain variable region having a sequence at least 95% identical to SEQ ID NO: 27 and a heavy chain variable region having a sequence at least 95% identical to SEQ ID NO: 29. In a preferred embodiment the antibody comprises a heavy chain variable region having a sequence at least 98% identical to SEQ ID NO: 29 and a light chain variable region having a sequence at least 98% identical to SEQ ID NO: 27.

In a further embodiment the antibody comprises a heavy chain variable region having a sequence at least 95% identical to SEQ ID NO: 33. In a further embodiment the antibody comprises a heavy chain variable region having a sequence at least 98% identical to SEQ ID NO: 33. In a further embodiment the antibody comprises a light chain variable region having a sequence at least 95% identical to SEQ ID NO: 31. In a further embodiment the antibody comprises a light chain variable region having a sequence at least 98% identical to SEQ ID NO: 31. In a further embodiment the antibody has a heavy chain variable region having a sequence at least 95% identical to SEQ ID NO: 33 and a light chain variable region having a sequence at least 95% identical to SEQ ID NO: 31. In a preferred embodiment the antibody has a heavy chain variable region having a sequence at least 98% identical to SEQ ID NO: 33 and a light chain variable region having a sequence at least 98% identical to SEQ ID NO: 31.

As is known to the person skilled in the art, antibodies contain multiple regions including framework regions. Deletion or addition of amino acids in the framework regions is unlikely to affect the ability of the antibody to bind to its target. On the other hand, mutations in the CDRs are considerably more likely to affect the ability of an antibody to bind to a target. Thus, variant antibodies bind to an epitope having an amino acid sequence of SEQ ID NO: 1 or 2 or have CDRs which are identical to the CDRs of the 12A7 or 4B4 antibodies. Variant antibodies of the invention may have framework regions which differ in sequence quite significantly from those described in SEQ ID NO: 27, 29, 31 or 33. Where an antibody of the invention comprises a heavy chain variable region having a sequence at least 85%, 90%, 95%, 98%, 99% or 100% identical to SEQ ID NO: 29, the antibody further comprises 12A7 CDRH1, 12A7 CDRH2 and 12A7 CDRH3. It is understood by the person skilled in the art that, since target binding specificity is determined by the CDRs, an antibody comprising the CDRs of 12A7 may still bind to Mcm5 even if the remainder of the antibody sequence is quite variable. For this reason where the antibody comprises 12A7 CDRH1, 12A7 CDRH2 and 12A7 CDRH3 the antibody preferably comprises a heavy chain variable region having a sequence at least 90% identical to SEQ ID NO: 29. In a more preferred embodiment the antibody of the invention comprises 12A7 CDRH1, 12A7 CDRH2 and 12A7 CDRH3 and comprises a heavy chain variable region having a sequence at least 95% identical to SEQ ID NO:29.

Where an antibody of the invention comprises a heavy chain variable region having a sequence at least 85%, 90%, 95%, 98%, 99% or 100% identical to SEQ ID NO: 33, the antibody further comprises 4B4 CDRH1, 4B4 CDRH2 and 4B4 CDRH3. It is understood by the person skilled in the art that, since target binding specificity is determined by the CDRs, an antibody comprising the CDRs of 4B4 may still bind to Mcm5 even if the remainder of the antibody sequence is quite variable. For this reason where the antibody comprises 4B4 CDRH1, 4B4 CDRH2 and 4B4 CDRH3 the antibody preferably comprises a heavy chain variable region having a sequence at least 90% identical to SEQ ID NO: 33. In a more preferred embodiment the antibody of the invention comprises 4B4 CDRH1, 4B4 CDRH2 and 4B4 CDRH3 and comprises a heavy chain variable region having a sequence at least 95% identical to SEQ ID NO: 33.

Where an antibody of the invention comprises a light chain variable region having a sequence at least 85%, 90%, 95%, 98%, 99% or 100% identical to SEQ ID NO: 27, the antibody further comprises 12A7 CDRL1, 12A7 CDRL2 and 12A7 CDRL3. It is understood by the person skilled in the art that, since target binding specificity is determined by the CDRs, an antibody comprising the CDRs of 12A7 may still bind to Mcm5 even if the remainder of the antibody sequence is quite variable. For this reason where the antibody comprises 12A7 CDRL1, 12A7 CDRL2 and 12A7 CDRL3 the antibody preferably comprises a light chain variable region having a sequence at least 90% identical to SEQ ID NO: 27. In a more preferred embodiment the antibody of the invention comprises 12A7 CDRL1, 12A7 CDRL2 and 12A7 CDRL3 and comprises a light chain variable region having a sequence at least 95% identical to SEQ ID NO: 27.

Where an antibody of the invention comprises a light chain variable region having a sequence at least 85%, 90%, 95%, 98%, 99% or 100% identical to SEQ ID NO: 31, the antibody further comprises 4B4 CDRL1, 4B4 CDRL2 and 4B4 CDRL3. It is understood by the person skilled in the art that, since target binding specificity is determined by the CDRs, an antibody comprising the CDRs of 4B4 may still bind to Mcm5 even if the remainder of the antibody sequence is quite variable. For this reason where the antibody comprises 4B4 CDRL1, 4B4 CDRL2 and 4B4 CDRL3 the antibody preferably comprises a heavy chain variable region having a sequence at least 90% identical to SEQ ID NO: 31. In a more preferred embodiment the antibody of the invention comprises 4B4 CDRL1, 4B4 CDRL2 and 4B4 CDRL3 and comprises a heavy chain variable region having a sequence at least 95% identical to SEQ ID NO: 31.

In a further embodiment of the invention the antibody of the invention comprises:
(i) 12A7 CDRH1, 12A7 CDRH2 and 12A7 CDRH3;
(ii) a heavy chain variable region having a sequence at least 95% identical to SEQ ID NO: 29;
(iii) 12A7 CDRL1, 12A7 CDRL2 and 12A7 CDRL3; and
(iv) a light chain variable region having a sequence at least 95% identical to SEQ ID NO: 27.

In a further embodiment the antibody of the invention comprises:
(i) 4B4 CDRH1, 4B4 CDRH2 and 4B4 CDRH3;
(ii) a heavy chain variable region having a sequence at least 95% identical to SEQ ID NO: 33;
(iii) 4B4 CDRL1, 4B4 CDRL2 and 4B4 CDRL3; and
(iv) a heavy chain variable region having a sequence at least 95% identical to SEQ ID NO: 31.

An antibody having a heavy chain variable sequence identical to SEQ ID NO: 29 and a light chain variable sequence identical to SEQ ID NO: 27 may be referred to as antibody 12A7. An antibody having a heavy chain variable sequence identical to SEQ ID NO: 33 and a light chain variable sequence identical to SEQ ID NO: 31 may be referred to as an antibody 4B4.

In some embodiments the variant antibody of the invention will compete for binding to Mcm5 with the 12A7 antibody. Similarly, in some embodiments of the invention the variant antibody of the invention will compete for binding to Mcm5 with the 4B4 antibody. For the purposes of the present invention an antibody is ' at least *90%, 95%, 98%, 99% or 100% identical'* to a second antibody if the sequences have at least 90%, 95%, 98%, 99% or 100% identity when assessed using ClustalW (Thompson *et al*., 1994) with the following parameters:
Pairwise alignment parameters -Method: accurate, Matrix:PAM, Gap open penalty: 10.00, Gap extension penalty: 0.10;
Multiple alignment parameters -Matrix: PAM, Gap open penalty: 10.00, %identity for delay: 30, Penalize end gaps: on, Gap separation distance: 0, Negative matrix: no, Gap extension penalty: 0.20, Residue-specific gap penalties: on, Hydrophilic gap penalties: on, Hydrophilic residues: GPSNDQEKR.

It is well within the knowledge of the person skilled in the art how to make variant antibodies which bind to Mcm5. Such variant antibodies may comprise 1, 2, 3, 4, 5, 6, 7, 8, 9, 10, 15, 20, 25, 50 or 100 substitution or deletion mutations compared to SEQ ID NOs: 27, 29, 31 or 33. *'Deletion'* variant antibodies may comprise the deletion of 1, 2, 3, 4, 5 or more amino acids or, in some cases, the deletion of entire regions of SEQ ID NOs: 27, 29, 31 or 33. *'Substitution'* variants may comprise the replacement of 1, 2, 3, 4, 5 or more amino acids with the same number of new amino acids.

Preferably, the variant antibodies comprise sequences differing from SEQ ID NOs: 27, 29, 31 or 33 by conservative amino acid substitutions (optionally only by conservative amino acid substitutions). The skilled person is well aware that such conservative substitutions are unlikely to alter the binding properties of an antibody.

### Antibodies that compete with antibodies of the invention

The disclosure further provides an antibody that competes with an antibody of the invention which:
(i) binds to a polypeptide having an amino acid sequence of SEQ ID NO: 1 or 2.
(ii) comprises at least one CDR selected from the group consisting of 12A7 CDRH1, 12A7 CDRH2, 12A7 CDRH3, 12A7 CDRL1, 12A7 CDRL2, and 12A7 CDRL3, or 4B4 CDRH1, 4B4 CDRH2, 4B4 CDRH3, 4B4 CDRL1, 4B4 CDRL2, and 4B4 CDRL3.

Preferably the disclosure provides an antibody that competes with an antibody comprising 12A7 CDRH1, 12A7 CDRH2, 12A7 CDRH3, 12A7 CDRL1, 12A7 CDRL2, and 12A7 CDRL3 or an antibody comprising 4B4 CDRH1, 4B4 CDRH2, 4B4 CDRH3, 4B4 CDRL1, 4B4 CDRL2, and 4B4 CDRL3. Even more preferably the disclosure provides an antibody that competes with an antibody comprising a heavy chain variable region having a sequence 100% identical to SEQ ID NO: 29 or 33 and a light chain variable region having a sequence 100% identical to SEQ ID NO: 27 or 31. The disclosure provides an antibody that competes with antibody 12A7 or antibody 4B4.

It is within the abilities of the person skilled in the art, to determine whether an antibody competes with another antibody of the invention.

For example, an ELISA assay may be performed by immobilising the reference antibody, such as a 4B4 or a 12A7 antibody, on an ELISA plate. The plate is then blocked with a suitable blocking agent. An excess of a second antibody is added (the second antibody is the antibody whose ability to compete with the 4B4 or 12A7 antibody is to be assessed for example an antibody of the invention that competes with 4B4 or 12A7). Mcm5 is added.

Following a suitable incubation period, the ELISA plate is washed and an Mcm5 detection agent is added to measure the amount of Mcm5 bound to the immobilised reference antibody. If this test is to be used to detect whether an antibody competes with 4B4, a suitable detection reagent is a labelled 12A7 antibody. Similarly if this test is to be used to detect whether an antibody competes with 12A7, a suitable detection reagent is a labelled 4B4 antibody. The concentration at which 50% inhibition occurs is known as the Ki. An antibody that competes with 12A7 or 4B4 may bind with a Ki 2-fold, 5-fold, 10-fold, 50-fold or 100-fold lower than an antibody which does not bind to Mcm5.

An antibody that competes with an antibody of the invention may be an antibody that binds to the same epitope (for example SEQ ID NO: 1 or SEQ ID NO: 2). It is possible to determine to which epitope an antibody binds by performing an experiment in which the target protein, such as Mcm5, is digested into fragments (for example see Example 3 below). The affinity with which the 12A7 and 4B4 antibodies to bind to each of the fragments can then be determined. Once the fragment (representing the epitope), to which the first antibody binds with a reasonable affinity, has been determined, the epitope may be synthesised. It is then possible to determine whether a second antibody is capable of binding to the same epitope by measuring the binding affinity of that second antibody to the fragment.

### Labelled antibodies

An antibody of the invention may be conjugated to a label (for example Europium³⁺ or Horseradish Peroxidase). The label may be directly, attached or may be attached via a linker (such as Adipic Acid Dihyrazide (ADH)).

The label may be attached by chemical conjugation. Methods of conjugating labels to antibodies are known in the art. For example, carbodiimide conjugation (Bauminger & Wilchek (1980) Methods Enzymol. 70, 151-159) may be used to conjugate labels to antibodies. Other methods for conjugating a label to an antibody can also be used. For example, sodium periodate oxidation followed by reductive alkylation of appropriate reactants can be used, as can glutaraldehyde cross-linking. However, it is recognised that, regardless of which method of producing a conjugate of the invention is selected, a determination must be made that the conjugated antibody maintains its targeting ability and that the conjugated label maintains its function.

In a preferred embodiment the antibody of the invention is labelled by conjugated to Europium. This may be achieved using an EG&G Wallac DELFIA^{(R)} Eu-labelling kit and following the manufacturer's protocol.

### Compositions

In further aspects of the invention, compositions are provided comprising an antibody of the invention. Such compositions may further comprise suitable stabilising agents that are capable of stabilising the antibody in solution. For example, the composition may comprise a buffer selected from the group consisting of TAPS (3-{[tris(hydroxymethyl)methyl]amino}propanesulphonic acid), Bicine (N,N-bis(2-hydroxyethyl)glycine), Tris (tris(hydroxymethyl)methylamine), Tricine (N-tris(hydroxymethyl(methylglycine), TAPSO (3-[N-Tris(hydroxymethyl)methylamino]-2-hydroxypropanesulphonic acid), HEPES (4-2-hydroxyethyl-1-piperazineethanesulphonic acid), phosphate buffer saline and MOPS (3-(N-morpholino)propanesulphonic acid. In a preferred embodiment the composition comprises phosphate-buffered saline at pH 7.6. In a further preferred embodiment the composition comprises phosphate-buffered saline with between 0.01% and 0.09% sodium azide. Preferably, the composition comprises agents suitable for storing the antibodies of the invention at room temperature or at a temperature less than or equal to 15°C, 10°C, 7°C, 5°C, 0°C, -10°C, or -25°C. In a preferred embodiment the composition comprises agents suitable for storing the antibodies of the invention at a temperature between 2°C and 8°C. In a further embodiment, the composition comprises a stabilising agent such as a sugar, for example lactose or trehalose.

If the antibody of the invention is conjugated, for example to a Europium label, it is advantageous to include a 7.5% purified BSA stabiliser in the composition.

### Nucleic acids and Vectors

A polynucleotide may comprise a nucleic acid sequence that encodes any antibody as described herein. The term *"nucleic acid molecule"* and *"polynucleotide"* are used interchangeably herein and refer to a polymeric form of nucleotides of any length, deoxyribonucleotides, ribonucleotides, or analogs thereof.

A nucleic acid which *"encodes"* a selected polypeptide is a nucleic acid molecule which is transcribed (in the case of DNA) and translated (in the case of mRNA) into a polypeptide *in vivo* when placed under the control of appropriate regulatory sequences. The boundaries of the coding sequence are determined by a start codon at the 5' (amino) terminus and a translation stop codon at the 3' (carboxy) terminus.

For example, a composition of the invention may comprise a nucleic acid that encodes an antibody comprising a variable region having a sequence at least 98% identical to SEQ ID NO: 29 or 33, and an antibody comprising a light chain variable region having a sequence at least 98% identical to SEQ ID NO: 27 or 31. A polynucleotide of the invention may encode both a polypeptide having a sequence at least 98% identical to SEQ ID NO: 29 and a polypeptide having a sequence at least 98% identical to SEQ ID NO: 27. Alternatively a polynucleotide of the invention may encode both a polypeptide having a sequence at least 98% identical to SEQ ID NO: 33 and a polypeptide having a sequence at least 98% identical to SEQ ID NO: 31.

An example composition of the invention comprises or consists of a nucleic acid sequence at least 98%, 99% or 100% identical to SEQ ID NO: 28 or 32 and a nucleic acid sequence at least 98%, 99% or 100% identical to SEQ ID NO: 30 or 34. The polynucleotide of the disclosure comprises or consists of a nucleic acid sequence at least 85%, 90%, 95%, 98%, 99% or 100% identical to SEQ ID NO: 28, 30, 32 or 34. Optionally the polynucleotide of the invention comprises a polynucleotide sequence having at least 98% identity to SEQ ID NO: 28 and a polynucleotide sequence having at least 98% identity to SEQ ID NO: 30. Optionally the polynucleotide of the invention comprises a polynucleotide sequence having at least 98% identity to SEQ ID NO: 32 and a polynucleotide sequence having at least 98% identity to SEQ ID NO: 34.

An antibody of the invention may thus be produced from a polynucleotide which encodes, and is capable of expressing, it. Where the antibody comprises two or more chains, a polynucleotide may encode an antibody light chain, and an antibody heavy chain or both. Two polynucleotides may be provided, one of which encodes an antibody light chain and the other of which encodes the corresponding antibody heavy chain. Such a polynucleotide or pair of polynucleotides may be expressed together such that an antibody of the invention is generated.

Polynucleotides of the invention can be synthesised according to methods well known in the art, as described by way of example in Sambrook et al (1989, Molecular Cloning - a laboratory manual; Cold Spring Harbor Press).

The invention further provides a vector (such as a plasmid or recombinant viral vector) comprising the polynucleotide of the invention. Typically the vector further comprises a control sequence operably linked to the inserted sequence, thus allowing for expression of the antibody of the invention *in vivo.* Preferably the vector of the invention further comprises appropriate initiators, promoters, enhances and other elements which may be necessary and which are positioned in the correct orientation, in order to allow for expression of a polypeptide of the invention.

### Hybridoma cell lines

The present invention further provides a hybridoma cell line capable of producing an antibody of the invention. Such a hybridoma cell line will produce the antibody of the invention when maintained in suitable culture conditions.

It is well within the ability of the person skilled in the art to develop a hybridoma expressing an antibody of the invention. This may be performed by immunising a mammal such as a mouse, rabbit or guinea pig, with an antigen (for example Mcm5). It may be beneficial to include an adjuvant such as Freund's complete adjuvant. The spleen cells of the immunised mammal are removed and fused with myeloma cells to form hybridoma lines which are immortal given appropriate conditions and which secrete antibodies. The hybridomas are separated into single clones and the antibodies secreted by each clone are evaluated for their binding ability to Mcm5 protein.

### Host cell lines

The invention also provides host cells comprising the nucleic acids or vectors of the invention. Such host cells include cells that have been modified to express an antibody of the invention. Such host cells may be eukaryotic cells or prokaryotic cells. For example, the host cells may be mammalian cells, insect cells, or bacterial cells. Particular examples of host cells that may be modified by insertion of vectors or nucleic acids of the invention include mammalian HEK293T, CHO, HeLa, NS0 and COS cells. Alternatively bacterial cells such as *E.coli* may be used.

Such host cells may be cultured using routine methods to produce an antibody of the invention.

### Antibody production

Antibodies of the invention may be produced using any method. In one embodiment, the antibodies are produced directly from a hybridoma cell line. In a further embodiment of the invention, the antibodies are produced recombinantly.

To produce an antibody from a hybridoma cell line, the cell line can be cultivated *in vitro,* for example in a vessel such as a cell culture flask or a fermenter, and the antibody can be isolated from the vessel using conventional techniques known in the art. For the purposes of the present invention, an antibody will be considered to have been *"produced by a hybridoma cell line"* if the antibody is derived from an antibody which was produced by the hybridoma cell line. For example, it is well within the abilities of the person skilled in the art to obtain an antibody from a hybridoma cell line, sequence the amino acid sequence of the antibody, and transfect cells such as CHO cells or *E.coli* cells with vectors encoding the amino acid sequence of the antibody. Such antibodies can be considered to have been *"derived"* from an antibody which was produced by the hybridoma cell line. In one particular embodiment, antibodies of the invention are produced directly from the hybridoma cell line itself.

Alternatively antibodies of the invention may be produced using recombinant techniques. The sequence of an antibody of the invention may be used to transfect a host cell line of the invention. The cell lines may be grown in a flask or a fermenter, and the antibody expressed by the cell can be isolated and purified using standard techniques (such as affinity chromatography).

Once purified the antibodies may then be conjugated to a radio label or immobilised to a solid support. An example of a suitable solid support is an ELISA plate.

### Kits and methods of determining the concentration of Mcm5 in a sample.

The antibodies of the invention may be used as part of a kit. For example, the antibodies may be part of a kit for use in detecting the concentration of Mcm5 in a sample, for example a sample from a human, this can allow detection of cancer in a subject from which the sample is taken.

Alternatively the antibodies of the invention may be used in a method of detecting the concentration or amount of Mcm5 in a sample (also referred to as an Mcm5 detection method of the invention). Preferably such a method comprises the following steps:
(i) providing an immobilised first antibody which is an antibody of the invention immobilised to a solid support;
(ii) exposing the sample to the immobilised first antibody such that Mcm5 in the sample may bind to the immobilised antibody to provide immobilised Mcm5;
(iii) providing a labelled second antibody which is an antibody of the invention conjugated to a label;
(iv) exposing the immobilised Mcm5 to the labelled second antibody such that the labelled second antibody may bind to the Mcm5; and
(v) detecting the concentration of the labelled second antibody.

Preferably there is a wash step between steps (iv) and (v) to ensure that the second antibody whose concentration is detected in step (v) bound to the Mcm5 in step (iv). Optionally there is a further wash step after step (i) to remove excess first antibody and/or after step (ii) to remove excess Mcm5. Optionally the wash step is performed by exposing the solid support to a wash buffer.

In one embodiment the antibodies present in a kit of the invention or used in a method of the invention are suitable for detecting a urological cancer such as prostate cancer or bladder cancer.

Preferably a method of detecting the concentration or amount of Mcm5 in a sample uses, or a kit of the invention comprises, two antibodies of the invention which do not compete with one another (such as the 4B4 and 12A7 antibodies) and preferably bind to different epitopes of the antigen of interest. This can be determined by performing an ELISA, such as that described above. One of the two antibodies may be immobilised onto an ELISA plate. The plate is then blocked with a suitable blocking agent. An excess of the other antibody is then added (the antibody whose ability to compete with the first antibody is to be assessed). The target antigen, such as Mcm5, is then added. Following a suitable incubation period, the ELISA plate is washed and a detection agent capable of binding to the target antigen is added to measure the amount of the target antigen bound to the immobilised antibody. If the two antibodies compete the amount of the target antigen bound to the immobilised antibody will be lower than if the antibodies do not compete (and, optionally, bind to different epitopes). The concentration at which 50% inhibition occurs is known as the Ki. In a preferred embodiment an antibody that competes with another antibody binds with a Ki 2-fold, 5-fold, 10-fold, 50-fold or 100-fold lower than an antibody which does not compete.

In a preferred embodiment an Mcm5 detection method of the invention uses, or a kit of the invention comprises, a first antibody of the invention and a second antibody of the invention. The first antibody may bind (optionally bind specifically) to SEQ ID NO: 1 and the second antibody may bind (optionally bind specifically) to SEQ ID NO: 2. The first antibody may comprise CDRs of 12A7 CDRH1, 12A7 CDRH2, 12A7 CDRH3, 12A7 CDRL1, 12A7 CDRL2, and 12A7 CDRL3. The second antibody may comprise CDRs of 4B4 CDRH1, 4B4 CDRH2, 4B4 CDRH3, 4B4 CDRL1, 4B4 CDRL2 and 4B4 CDRL3. Preferably the first antibody comprises 12A7 CDRH1, 12A7 CDRH2, 12A7 CDRH3, 12A7 CDRL1, 12A7 CDRL2, and 12A7 CDRL3. Preferably the second antibody comprises 12A7 CDRH1, 12A7 CDRH2, 12A7 CDRH3, 12A7 CDRL1, 12A7 CDRL2, and 12A7 CDRL3. The first antibody may comprise a heavy chain variable region having a sequence at least 85%, 90%, 95%, 98%, 99% or 100% identical to SEQ ID NO: 29 and a light chain variable region sequence having a sequence at least 85%, 90%, 95%, 98%, 99% or 100% identical to SEQ ID NO: 27. The second antibody may comprise a heavy chain variable region having a sequence at least 85%, 90%, 95%, 98%, 99% or 100% identical to SEQ ID NO: 33 and a light chain variable region sequence having a sequence at least 85%, 90%, 95%, 98%, 99% or 100% identical to SEQ ID NO: 31.

A DELFIA detection system may be used in an Mcm5 detection method of the invention. In such embodiments the first antibody and the second antibody preferably bind different epitopes. In such embodiments one of the antibodies (the first or the second monoclonal antibody) can be immobilised on a plate. A sample to be tested can be added to the plate. The other antibody (the first or the second antibody which was not immobilised to the plate) can then be added. The other antibody should be conjugated to a detection agent such as a Europium label. Time-resolved fluorescent spectroscopy can then be used to determine whether the target antigen is present (by measuring the amount of the labelled antibody that is present).

Accordingly, in one embodiment of the invention, a kit of the invention comprises a first or second antibody of the invention which is conjugated to a radiolabel, for example Europium. Any method may be used to conjugate the first monoclonal antibody or the second monoclonal antibody to a Europium label. Kits for performing such labelling are available. For example, the first or second monoclonal antibody may be conjugated to Europium using an Eu-NI-ITC chelate.

In such embodiments a kit of the invention may comprise further components for use in a DELFIA detection system. Such further components may include a DELFIA enhancement solution or a DELFIA detection solution

### Definitions

The terms *"comprises* " means *"includes".* Thus, the word *"comprises"* and variations such as *"comprise* ", and *"comprising"* will be understood to imply the inclusion of a stated compound or composition or step, or group of compounds or steps, but not to the exclusion of any other compounds, composition, steps or groups thereof.

**Table 1: Sequences**

| SEQ ID NO | Nucleotide/Polypeptide |
|---|---|
| 1 | WDETKGE (epitope to which antibody 12A7 binds) |
| 2 | DDRVAIH (epitope to which antibody 4B4 binds |
| 3 | 12A7 light chain CDR 1 polypeptide sequence |
| 4 | 12A7 light chain CDR 1 nucleotide sequence |
| 5 | 12A7 light chain CDR 2 polypeptide sequence |
| 6 | 12A7 light chain CDR 2 nucleotide sequence |
| 7 | 12A7 light chain CDR 3 polypeptide sequence |
| 8 | 12A7 light chain CDR 3 nucleotide sequence |
| 9 | 12A7 heavy chain CDR 1 polypeptide sequence |
| 10 | 12A7 heavy chain CDR 1 nucleotide sequence |
| 11 | 12A7 heavy chain CDR 2 polypeptide sequence |
| 12 | 12A7 heavy chain CDR 2 nucleotide sequence |
| 13 | 12A7 heavy chain CDR 3 polypeptide sequence |
| 14 | 12A7 heavy chain CDR 3 nucleotide sequence |
| 15 | 4B4 light chain CDR 1 polypeptide sequence |
| 16 | 4B4 light chain CDR 1 nucleotide sequence |
| 17 | 4B4 light chain CDR 2 polypeptide sequence |
| 18 | 4B4 light chain CDR 2 nucleotide sequence |
| 19 | 4B4 light chain CDR 3 polypeptide sequence |
| 20 | 4B4 light chain CDR 3 nucleotide sequence |
| 21 | 4B4 heavy chain CDR 1 polypeptide sequence |
| 22 | 4B4 heavy chain CDR 1 nucleotide sequence |
| 23 | 4B4 heavy chain CDR 2 polypeptide sequence |
| 24 | 4B4 heavy chain CDR 2 nucleotide sequence |
| 25 | 4B4 heavy chain CDR 3 polypeptide sequence |
| 26 | 4B4 heavy chain CDR 3 nucleotide sequence |
| 27 | 12A7 full light chain variable region sequence (polypeptide) |
| 28 | 12A7 full light chain variable region sequence (nucleotide) |
| 29 | 12A7 full heavy chain variable region sequence (polypeptide) |
| 30 | 12A7 full heavy chain variable region sequence (nucleotide) |
| 31 | 4B4 full light chain variable region sequence (polypeptide) |
| 32 | 4B4 full light chain variable region sequence (nucleotide) variable region |
| 33 | 4B4 full heavy chain variable region sequence (polypeptide) |
| 34 | 4B4 full heavy chain variable region sequence (nucleotide) |
| 35 | Mcm5 polypeptide sequence |
| 36 | Mcm5 polynucleotide sequence |

### Examples

### Cloning of Mcm5

### Stage I: Cloning Strategy

1. A verified IMAGE clone containing the human *MCM5* cDNA was obtained and used as a PCR template in order to amplify a 648bp fragment of DNA. The forward PCR primer encoded n initiation codon as well as a modified unpatented poly-Histidine tag (HQ)₄.
2. The PCR fragment was cloned into the inducible expression *E. coli* vector pTRC99a using a double restriction site strategy. This vector allows inducible expression directed by the hybrid *trp*/*lac "trc"* promoter. Downstream is a *lacZ* ribosome binding site (RBS) that is situated at an optimized distance from an initiation ATG codon, which is supplied by the *Nco*I cloning site.

### Stage IIA: Expression and Purification (small scale - 10ml)

3. The recombinant plasmid containing the Mcm5 fragment was transformed into BL21 *E. coli* (nοn-λDE3 to ensure high yields of expressed protein.
4. Several small volume *E. coli* cultures were induced with IPTG. Cells will be lysed in SDS loading buffer and total protein separated by SDS-PAGE. High expressing clones will be identified by Western blotting using a mouse monoclonal supplied by Urosens.

### Stage IIB: Expression and Purification (large scale - 500ml)

*5.* The identified clone with high levels of recombinant protein was cultured at the 0.5L scale and induced with IPTG. Cells were pelleted and stored at -70°C. Cells were disrupted in a denaturing lysis buffer described by Stoeber et al. J Natl Cancer Inst 2002 94 1071-9.
6. His-tagged recombinant protein was affinity purified under denaturing conditions on an immobilized metal affinity (IMAC) chromatography column. Protein were eluted using an acidic buffer and dialysed against storage buffer (PBS containing 0.3 SDS)
7. Eluted protein was analyzed and quantified by SDS-PAGE and western blotting using a mouse monoclonal supplied by Urosens.

### Example 1 - MAb screening ELISA

Protein A purified MAbs were obtained from the Welcome/CRUK Institute for Cancer and Developmental Biology. These antibodies were screened for their ability to bind to Mcm5.

An ELISA of recombinant His6-tagged Mcm5 was performed using Ni-NTA His-Sorb 96 well microtitre coated plate wells (Qiagen). The Ni-NTA coated plates, pre-blocked with BSA, ensure an orientated presentation of the 6x His tagged Mcm through Nickel affinity binding to the spacer in comparison to the random presentation of the Mcm5 to the well that would be achieved by passive adsorption plate coating. The Mcm5 at a concentration of 625 ng/ml was Nickel affinity immobilised to Ni-NTA His-Sorb surface (Qiagen) of a 96 well plate at 200 µl/well resulting in 125 ng *hs*Mcm5/well. This was done following the manufacturers instructions to apply a His-6 tagged protein or peptide to the plate wells at a titre>100 ng/well. This was done to ensure that the protein or peptide is not limiting to Ab detection. The screening of the MAbs for development of a Mcm5 IFMA was performed with an enzyme linked immunosorbent assay (ELISA) of Mcm5. To screen MAbs, supernatants from hybridoma cultures expressing the MAbs were incubated in the microtitre wells at RT for 3 h and then washed away with an automated 96 well plate washer. The Mcm5 Ag-Ab complexes of the MAbs were detected by a colorimetric ELISA using goat anti-mouse IgG conjugated to HRP, TMB reagent and 1M H₂SO₄ assay stop solution, for the measurement of light absorbance at 450 nanometres (Abs 450 nm). An absence of Non-specific binding (NSB) of goat anti-mouse HRP conjugate to the Ni-NTA His-Sorb surface and Mcm5 was excluded by performing the ELISA on wells free from MAbs. The degree of NSB of the mouse MAbs being screened to the Ni-NTA coated polystyrene wells was established by performing the ELISA in wells with the omission of Mcm5 wells in parallel to performing the ELISA in wells with the Nickel affinity immobilisation of Mcm5 to the microtitre wells.

The performance in the colorimetric ELISA of a mouse MAb in the media samples taken from the hybridomas (Table 2) was used for selection of the highlighted clones, 12A7 and 4B4. The selected MAb hybridomas were grown up to produce IgG for purification from the culture supernatant using solid-phase protein-A separation according to the manufacturer's instructions (Amersham Pharmacia Biotech Little Chalfont Buckinghamshire, UK). The 12A7 MAb gave rise to the greatest response to the Mcm5.

**Table 2: hybridoma MAb screening ELISA Abs 450-650 results**

| **Mcm5 MAb** | **Mcm5 well response** | **NSB well response** | **Mcm5 specific response** |
|---|---|---|---|
| **12A7** | 1.404 | 0.421 | **0.983** |
| **4B4** | 1.622 | 1.040 | **0.582** |

| | | | |
|---|---|---|---|
| MAb relative affinities | | | |

Relative affinities of the protein A purified MAbs were determined by normalisation of Ab concentrations and application of the MAbs to the colorimetric screening assay under Ag-limiting conditions. Ag limiting conditions were determined by linear titration of Mcm5 across the Ni-NTA His-Sorb microtitre plate wells. An observed maximal Abs 450 nm of <3.000 is indicative of Ag limiting conditions, because the maximum Abs 450 reading obtainable when Ag is non-limiting is 4.000. The Ag titration was performed using rabbit Mcm5 PAb (101) and Goat anti Rabbit HRP to measure ELISA response. A concentration that gave 25 ng of Mcm5 available for binding to each Ni-NTA well was limiting to the ELISA Abs 450 response of the PAb. The rabbit PAb was chosen to determine Ag-limiting conditions for investigation of the mouse MAbs, as the full array of Mcm5 epitopes are bound by the PAb. This ensured that the MAbs could be investigated in combined incubation studies to determine the degree of epitope competition.

The concentration of IgG in the protein-A purified MAb solutions was determined by Abs 280 nm measurement in a spectrophotometer and MAb concentrations were normalised by dilution in PBS. The 50% maximum binding concentration was then determined by a linear dilution of the MAbs applied to an ELISA of Nickel affinity Mcm5 wells under Mcm5 Ag-limiting conditions. To demonstrate the plateau of the binding signal to the 25 ng/well *hs*Mcm5rf on the plate the MAb 7A3 was run in the ELISA over a greater concentration range. The 50% maximum binding response for 12A7 was obtained at a concentration of 20 ng/well. 12A7 is shown by its 50% maximum binding concentration to demonstrate the highest relative affinity to *hs*Mcm5. (Table 3).

**Table 3: MAb titrations against limiting 25ng/well antigen couples to Ni-NTA**

| | MAb | |
|---|---|---|
| nb/well MAb | 4B4 | 12A7 |
| 0 | 0.086 | 0.051 |
| 0.25 | 0.108 | 0.189 |
| 2.5 | 0.464 | 0.762 |
| 25 | 0.966 | 1.804 |
| 500 | 1.848 | 2.808 |
| 1000 | 2.414 | 3.099 |

### Example 2 - Antibody epitope competition

For a two-site immunometric assay two MAbs that are targeted to distinct epitopes are required. In Ag limiting conditions a pair of MAbs competing for the same epitope will provide an ELISA Abs 450 nm measurement equal to the Abs 450 nm signal of the higher relative affinity Ab when it is incubated in the absence of the competing MAb, however, incubation of a pair of MAbs targeted to distinct epitopes will provide an Abs 450 measurement equal to the sum of both the MAbs individual Abs 450 nm responses to the limited Ag available provided there is an absence of steric hindrance. The MAbs 12A7 and 4B4 were normalised to the 50% maximum binding concentration of 20 ng/well of the highest relative affinity MAb 12A7. The MAbs at normalised concentration underwent an ELISA using both individual and paired Ab incubations on the 25 ng/well Mcm5 Ag limiting Ni-NTA plate. The highest Abs 450 nm signal measured from a combined incubation of a pair of the MAbs would be indicative of the most suitable MAbs for use in a two-site IFMA.

### Antibody epitope competition results

The Ab Ag epitope competition ELISA study results (Table 4) show that the MAb 4B4 is targeted to a distinct epitope compatible for two-site binding with the 12A7. The distinct epitope targeted by 4B4 is demonstrated by the Abs 450 response' being equal to the sum of both MAbs binding to the limiting Ag when 4B4 was incubated in parallel to the other MAbs.

**Table 4: Ab Ag epitope competition ELISA study results**

| **MAb 20 ng/well incubation** | **Mcm5 25ng/well Abs 450-650 response** |
|---|---|
| 12A7 | 1.375 |
| 4B4 | 1.244 |
| 12A7+4B4 | 2.693 |

The MAbs were incubated at a normalised concentration of 20 ng/well, the 50% maximum binding concentration of the MAb with the highest relative affinity 12A7. The optimal MAbs for two-site immunoassay development are highlighted in blue, and their ELISA Abs 450 responses highlighted in yellow.

### Example 3 - Epitope mapping

### Materials used:

| | |
|---|---|
| Microarray Content: | The sequence of antigen Mcm 5 (aa 367-582) was translated into 15 aa peptides with a peptide-peptide overlap |
| Samples: | Mouse monoclonal IgG antibodies 12A7 and 4B4 |
| Washing Buffer: | PBS, pH 7.4 with 0.05% Tween 20 (3x1 min after each assay) |
| Blocking Buffer: | Rockland blocking buffer MB-070 (30 min before the first assay) |
| Incubation Buffer: | PBS, pH 7.4 with 0.05% Tween 20 and 10% Rockland blocking buffer |
| Assay Conditions: | Antibody concentrations of 1 µg/ml and 10 µg/ml in incubation buffer; incubation for 16 h at 4°C and |
| Secondary Antibody: | Goat anti-mouse IgG (H+L) DyLight 680 antibody; |
| Control Antibodies: | Monoclonal anti-HA (12CA5)-DyLight680 (1:1000), monoclonal anti-FLAG(M2)-DyLight800 (1:500); staining in |
| Scanner: | LI-COR Odyssey Imaging System; scanning offset 0.8 mm, resolution 21 µm, scanning intensity red/green of 5/7 |

Pre-staining of one of the peptide microarrays was done with the secondary goat anti-mouse IgG (H+L) DyLight680 antibody at a dilution of 1:5000 to investigate background interactions with the antigen-derived peptides that could interfere with the main assays. Subsequent incubation of the peptide microarrays with mouse monoclonal IgG antibodies 12A7 and 4B4 at concentrations of 1 µg/ml and 10 µg/ml in incubation buffer was followed by staining with the secondary antibody and read-out at a scanning intensity of 5 (red). HA and Flag control peptides framing the peptide arrays were finally stained as internal quality control to confirm the assay quality and the peptide microarray integrity (scanning intensities red/green: 5/7).

Quantification of spot intensities and peptide annotation were done with PepSlide® Analyzer. A software algorithm breaks down fluorescence intensities of each spot into raw, foreground and background signal and calculates the standard deviation of foreground median intensities. Based on averaged foreground median intensities, intensity maps were generated and interactions in the peptide maps highlighted by an intensity color code with red for high and white for low spot intensities.

We further plotted averaged spot intensities of all assays with the antibody samples against the antigen sequence from the N- to the C-terminus to visualize overall spot intensities and signal to noise ratios. The intensity plots were correlated with peptide and intensity maps as well as with visual inspection of the microarray scans to identify peptides and epitopes that were recognized by the antibody samples. In case it was not clear if a certain amino acid contributed to antibody binding.

After 15 min pre-swelling in washing buffer and 30 min in blocking buffer, one of the peptide microarrays was initially incubated with the secondary goat anti-mouse IgG (H+L) DyLight680 antibody at a dilution of 1:5000 for 30 min at room temperature to analyze background interactions with the antigen-derived peptides. At a scanning intensity of 5, we did not observe any background due to non-specific binding of the secondary antibody. Data quantification with PepSlide® Analyzer was neither possible nor required, since the absence of any spot pattern hampered alignment of the microarray grid.

The peptide microarrays were incubated with mouse monoclonal antibody 12A7 at concentrations of 1 µg/ml and 10 µg/ml (top right). After each incubation, staining with the secondary goat anti-mouse IgG (H+L) DyLight680 antibody was followed by read-out at a scanning intensity of 5. We observed a strong and well-defined epitope-like spot pattern formed by a row of neighbored peptides with a consensus motif at excellent signal to noise ratios.

The final staining of the HA and Flag control peptides framing the peptide microarray gave rise to the expected and well-defined spot pattern and validated the overall peptide microarray integrity.

Data quantification was followed by generation of peptide and intensity maps as well as of intensity plots for the assays with mouse monoclonal antibody 12A7 at concentrations of 1 µg/ml and 10 µg/ml; the intensity plot of the latter was leveled to provide a clearer data overview. The signals were based on the epitope-like spot pattern observed in the microarray scan and attributed to peptides with the consensus motif WDETKGE.

The peptide microarrays were incubated with mouse monoclonal antibody 4B4 at concentrations of 1 µg/ml) and 10 µg/ml. After each incubation, staining with the secondary goat anti-mouse IgG (H+L) DyLight680 antibody was followed by read-out at a scanning intensity of 5. We observed two strong and well-defined epitope-like spot patterns formed by a row of neighbored peptides with consensus motifs at excellent signal to noise ratios. At an antibody concentration of 10 µg/ml the raw intensities of the epitope-like spot pattern were similar but the background interactions were slightly increased indicating a signal saturation of the antibody in the concentration range of 1 - 10 µg/ml. The final staining of the HA and Flag control peptides framing the peptide microarray gave rise to the expected and well-defined spot pattern and validated the overall peptide microarray integrity.

Data quantification was followed by generation of peptide and intensity maps as well as of intensity plots for the assays with mouse monoclonal antibody 4B4 at concentrations of 1 µg/ml and 10 µg/ml; the intensity plot of the latter was leveled to provide a clearer data overview. At an antibody concentration of 10 µg/ml the raw intensities of the epitope-like spot pattern were similar but the background interactions were slightly increased leading to reduced normalized signal intensities. The signals were based on the epitope-like spot patterns observed in the microarray scan and attributed to peptides with the consensus motifs DDRVAIH and WDETKGE.

### Sequence listing

SEQ ID NO: 1
   WDETKGE
SEQ ID NO: 2
   DDRVAIH
SEQ ID NO: 3
   QNLVQSNGNTY
SEQ ID NO: 4
   CAGAACCTTGTACAAAGTAATGGAAACACCTATTTA
SEQ ID NO: 5
   KVS
SEQ ID NO: 6:
   AAGTTTCCAA
SEQ ID NO: 7:
   SQSTRVPYT
SEQ ID NO: 8:
   TCTCAAAGTACACGTGTTCCGTACACA
SEQ ID NO: 9:
   GFSLSTSGMG
SEQ ID NO: 10:
   GGGTTTTCACTGAGCACTTCTGGTATGGGT
SEQ ID NO: 11:
   IFWDDDK
SEQ ID NO: 12:
   ATTTTCTGGGATGATGACAAG
SEQ ID NO: 13:
   ARRSDYNYYSMDY
SEQ ID NO: 14:
   GCGCGGCGAAGTGACTACAATTACTACTCTATGGACTAC
SEQ ID NO: 15:
   QDIGSS
SEQ ID NO: 16:
   CAGGACATTGGTAGTAGC
SEQ ID NO: 17:
   ATS
SEQ ID NO: 18:
   GCCACATCC
SEQ ID NO: 19:
   LQYASSPPT
SEQ ID NO: 20:
   CTACAATATGCTAGTTCTCCTCCGACG
SEQ ID NO: 21:
   GFTFSNYA
SEQ ID NO: 22:
   GGATTCACTTTCAGTAACTATGCC
SEQ ID NO: 23:
   ISRGGSYT
SEQ ID NO: 24:
   ATTAGTCGTGGTGGTAGTTACACC
SEQ ID NO: 25:
   ARHGYNYDDGAWFAN
SEQ ID NO: 26:
   GCAAGACATGGATATAATTACGACGACGGGGCCTGGTTTGCTAAC
SEQ ID NO: 27:
SEQ ID NO: 28:
SEQ ID NO: 29:
SEQ ID NO: 30:
SEQ ID NO: 31:
SEQ ID NO: 32:
SEQ ID NO: 33:
SEQ ID NO: 34:
SEQ ID NO: 35:
SEQ ID NO:36
   Homo sapiens minichromosome maintenance complex component 5 (MCM5), mRNA NCBI Reference Sequence: NM_006739.3

## Claims

1. A monoclonal antibody that specifically binds to Mcm5 which binds to an epitope having an amino acid sequence of
(i) SEQ ID NO: 2; or
(ii) SEQ ID NO: 1.

2. A monoclonal antibody that specifically binds to Mcm5 which comprises 4B4 CDRH1 which has a sequence of SEQ ID NO: 21, 4B4 CDRH2 which has a sequence of SEQ ID NO: 23, 4B4 CDRH3 which has a sequence of SEQ ID NO: 25, 4B4 CDRL1 which has a sequence of SEQ ID NO: 15, 4B4 CDRL2 which has a sequence of SEQ ID NO: 17, and 4B4 CDRL3 which has a sequence of SEQ ID NO: 19.

3. A monoclonal antibody that specifically binds to Mcm5 which comprises 12A7 CDRH1 which has a sequence of SEQ ID NO: 9, 12A7 CDRH2 which has a sequence of SEQ ID NO: 11, 12A7 CDRH3 which has a sequence of SEQ ID NO: 13, 12A7 CDRL1 which has a sequence of SEQ ID NO: 3, 12A7 CDRL2 which has a sequence of SEQ ID NO: 5, and 12A7 CDRL3 which has a sequence of SEQ ID NO: 7.

4. The antibody of any one of claims 1-3 which has an affinity for Mcm5 in the range of 1-10 nM.

5. The antibody of claim 1(ii) or claim 3 wherein the antibody comprises a heavy chain variable region having a sequence at least 98% identical to SEQ ID NO: 29, and the antibody comprises a light chain variable region having a sequence at least 98% identical to SEQ ID NO: 27.

6. The antibody of claim 1(i) or claim 2 wherein the antibody comprises a heavy chain variable region having a sequence at least 98% identical to SEQ ID NO: 33, and the antibody comprises a light chain variable region having a sequence at least 98% identical to SEQ ID NO: 31.

7. A composition comprising the antibody of any one of claims 1-6.

8. A composition comprising:
(i) a nucleic acid sequence at least 98%, 99%, or 100% identical to SEQ ID NO: 28 and a nucleic acid sequence at least 98%, 99%, or 100% identical to SEQ ID NO: 30; or
(ii) a nucleic acid sequence at least 98%, 99%, or 100% identical to SEQ ID NO: 32 and a nucleic acid sequence at least 98%, 99%, or 100% identical to SEQ ID NO: 34; or
(iii) a nucleic acid sequence of SEQ ID NO: 28 and a nucleic acid sequence of SEQ ID NO: 30; or
(iv) a nucleic acid sequence of SEQ ID NO: 32 and a nucleic acid sequence of SEQ ID NO: 34.

9. A host cell comprising the nucleic acid of claim 8, optionally wherein the host cell is a CHO cell or an *E.coli* cell.

10. A method of making an antibody comprising:
(i) growing a host cell of claim 9; and
(ii) isolating an antibody from the host cell.

11. The method of claim 10:
(i) further comprising a step of labelling the antibody by conjugating the antibody to a radio label; or
(ii) further comprising a step of immobilising the antibody to a solid support.

12. A kit comprising:
(i) a composition comprising a first antibody according to any one of claims 1(i), 2 or 6; and
(ii) a composition comprising a second antibody according to any one of claims 1(ii), 3 or 5.

13. A method of determining the concentration or amount of Mcm5 in a sample comprising the following steps:
(i) providing an immobilised first antibody which is an antibody of any one of claims 1-6 immobilised to a solid support;
(ii) exposing the sample to the immobilised first antibody such that Mcm5 in the sample may bind to the immobilised antibody to provide immobilised Mcm5;
(iii) providing a labelled second antibody which is an antibody of any one of claims 1-6 conjugated to a label;
(iv) exposing the immobilised Mcm5 to the labelled second antibody such that the labelled second antibody may bind to the Mcm5; and
(v) detecting the concentration of the labelled second antibody;
wherein the first antibody and the second antibody both bind to Mcm5 but the first antibody and the second antibody bind to different epitopes of Mcm5.

14. The kit of claim 12 or the method of claim 13 wherein the first antibody and the second antibody both bind to Mcm5 but the first antibody and the second antibody bind to different epitopes of Mcm5; and
(a)
(i) the first antibody binds to SEQ ID NO: 1 and the second antibody binds to SEQ ID NO: 2; or
(ii) the first antibody binds to SEQ ID NO: 2 and the second antibody binds to SEQ ID NO: 1; and/or
(b) the first antibody is immobilised to an ELISA plate; and/or
(c) the second antibody is immobilised to an ELISA plate; and/or
(d) the second antibody is conjugated to a radioactive label, optionally wherein the radioactive label is Europium ³⁺; and/or
(e) the first antibody is conjugated to a radioactive label, optionally wherein the radioactive label is Europium ³⁺.

15. The method of any one of claims 13-14 wherein the step of detecting the concentration of the labelled second antibody comprises immunofluorescent detection of Eu³⁺.

## Patentansprüche

1. Monoklonaler Antikörper, der spezifisch an Mcm5 bindet, der an ein Epitop mit einer Aminosäuresequenz von
(i) SEQ ID NO: 2; oder
(ii) SEQ ID NO: 1
bindet.

2. Monoklonaler Antikörper, der spezifisch an Mcm5 bindet, der 4B4-CDRH1 mit einer Sequenz von SEQ ID NO: 21, 4B4-CDRH2 mit einer Sequenz von SEQ ID NO: 23, 4B4-CDRH3 mit einer Sequenz von SEQ ID NO: 25, 4B4-CDRL1 mit einer Sequenz von SEQ ID NO: 15, 4B4-CDRL2 mit einer Sequenz von SEQ ID NO: 17 und 4B4-CDRL3 mit einer Sequenz von SEQ ID NO: 19 umfasst.

3. Monoklonaler Antikörper, der spezifisch an Mcm5 bindet, der 12A7-CDRH1 mit einer Sequenz von SEQ ID NO: 9, 12A7-CDRH2 mit einer Sequenz von SEQ ID NO: 11, 12A7-CDRH3 mit einer Sequenz von SEQ ID NO: 13, 12A7-CDRL1 mit einer Sequenz von SEQ ID NO: 3, 12A7-CDRL2 mit einer Sequenz von SEQ ID NO: 5 und 12A7-CDRL3 mit einer Sequenz von SEQ ID NO: 7 umfasst.

4. Antikörper nach einem der Ansprüche 1 bis 3, der eine Affinität für Mcm5 im Bereich von 1 bis 10 nM aufweist.

5. Antikörper nach Anspruch 1 (ii) oder Anspruch 3, wobei der Antikörper eine variable Region der schweren Kette mit einer Sequenz umfasst, die zumindest zu 98 % mit SEQ ID NO: 29 identisch ist, und wobei der Antikörper eine variable Region der leichten Kette mit einer Sequenz umfasst, die zumindest zu 98 % mit SEQ ID NO: 27 identisch ist.

6. Antikörper nach Anspruch 1 (i) oder Anspruch 2, wobei der Antikörper eine variable Region der schweren Kette mit einer Sequenz umfasst, die zumindest zu 98 % mit SEQ ID NO: 33 identisch ist, und wobei der Antikörper eine variable Region der leichten Kette mit einer Sequenz umfasst, die zumindest zu 98 % mit SEQ ID NO: 31 identisch ist.

7. Zusammensetzung, die den Antikörper nach einem der Ansprüche 1 bis 6 umfasst.

8. Zusammensetzung, die umfasst:
(i) eine Nukleinsäuresequenz, die zumindest zu 98 %, 99 % oder 100 % mit SEQ ID NO: 28 identisch ist, und eine Nukleinsäuresequenz, die zumindest zu 98 %, 99 % oder 100 % mit SEQ ID NO: 30 identisch ist; oder
(ii) eine Nukleinsäuresequenz, die zumindest zu 98 %, 99 % oder 100 % mit SEQ ID NO: 32 identisch ist, und eine Nukleinsäuresequenz, die zumindest zu 98 %, 99 % oder 100 % mit SEQ ID NO: 34 identisch ist; oder
(iii) eine Nukleinsäuresequenz von SEQ ID NO: 28 und eine Nukleinsäuresequenz von SEQ ID NO: 30; oder
(iv) eine Nukleinsäuresequenz von SEQ ID NO: 32 und eine Nukleinsäuresequenz von SEQ ID NO: 34.

9. Wirtszelle, die die Nukleinsäure nach Anspruch 8 umfasst, optional wobei die Wirtszelle eine CHO-Zelle oder eine *E.-coli*-Zelle ist.

10. Verfahren zum Herstellen eines Antikörpers, das umfasst:
(i) Züchten einer Wirtszelle nach Anspruch 9; und
(ii) Isolieren eines Antikörpers aus der Wirtszelle.

11. Verfahren nach Anspruch 10:
(i) das ferner einen Schritt des Markierens des Antikörpers durch Konjugieren des Antikörpers mit einer Radiomarkierung umfasst; oder
(ii) das ferner einen Schritt des Immobilisierens des Antikörpers an einem festen Träger umfasst.

12. Kit, der umfasst:
(i) eine Zusammensetzung, die einen ersten Antikörper nach einem der Ansprüche 1 (i), 2 oder 6 umfasst; und
(ii) eine Zusammensetzung, die einen zweiten Antikörper nach einem der Ansprüche 1 (ii), 3 oder 5 umfasst.

13. Verfahren zum Ermitteln der Konzentration oder Menge von Mcm5 in einer Probe, das die folgenden Schritte umfasst:
(i) Bereitstellen eines immobilisierten ersten Antikörpers, der ein Antikörper nach einem der Ansprüche 1 bis 6 ist, der an einem festen Träger immobilisiert ist;
(ii) Aussetzen der Probe gegenüber dem immobilisierten ersten Antikörper, so dass Mcm5 in der Probe an den immobilisierten Antikörper binden kann, um immobilisiertes Mcm5 bereitzustellen;
(iii) Bereitstellen eines markierten zweiten Antikörpers, der ein Antikörper nach einem der Ansprüche 1 bis 6 ist, der mit einer Markierung konjugiert ist;
(iv) Aussetzen des immobilisierten Mcm5 gegenüber dem markierten zweiten Antikörper, so dass der markierte zweite Antikörper an das Mcm5 binden kann; und
(v) Nachweisen der Konzentration des markierten zweiten Antikörpers;
wobei der erste Antikörper und der zweite Antikörper beide an Mcm5 binden, der erste Antikörper und der zweite Antikörper jedoch an unterschiedliche Epitope von Mcm5 binden.

14. Kit nach Anspruch 12 oder Verfahren nach Anspruch 13, wobei der erste Antikörper und der zweite Antikörper beide an Mcm5 binden, der erste Antikörper und der zweite Antikörper jedoch an unterschiedliche Epitope von Mcm5 binden; und
(a)
(i) der erste Antikörper an SEQ ID NO: 1 bindet und der zweite Antikörper an SEQ ID NO: 2 bindet; oder
(ii) der erste Antikörper an SEQ ID NO: 2 bindet und der zweite Antikörper an SEQ ID NO: 1 bindet; und/oder
(b) der erste Antikörper an einer ELISA-Platte immobilisiert ist; und/oder
(c) der zweite Antikörper an einer ELISA-Platte immobilisiert ist; und/oder
(d) der zweite Antikörper mit einer radioaktiven Markierung konjugiert ist, optional wobei die radioaktive Markierung Europium ³⁺ ist; und/oder
(e) der erste Antikörper mit einer radioaktiven Markierung konjugiert ist, optional wobei die radioaktive Markierung Europium ³⁺ ist.

15. Verfahren nach einem der Ansprüche 13 bis 14, wobei der Schritt des Nachweisens der Konzentration des markierten zweiten Antikörpers einen Nachweis von Eu³⁺ mittels Immunfluoreszenz umfasst.

## Revendications

1. Anticorps monoclonal qui se lie spécifiquement à Mcm5 qui se lie à un épitope ayant une séquence d'acides aminés de
(i) SEQ ID NO : 2 ; ou
(ii) SEQ ID NO : 1.

2. Anticorps monoclonal qui se lie spécifiquement à Mcm5 qui comprend 4B4 CDRH1 qui a une séquence de SEQ ID NO : 21, 4B4 CDRH2 qui a une séquence de SEQ ID NO : 23, 4B4 CDRH3 qui a une séquence de SEQ ID NO : 25, 4B4 CDRL1 qui a une séquence de SEQ ID NO : 15, 4B4 CDRL2 qui a une séquence de SEQ ID NO : 17 et 4B4 CDRL3 qui a une séquence de SEQ ID NO : 19.

3. Anticorps monoclonal qui se lie spécifiquement à Mcm5 qui comprend 12A7 CDRH1 qui a une séquence de SEQ ID NO : 9, 12A7 CDRH2 qui a une séquence de SEQ ID NO : 11, 12A7 CDRH3 qui a une séquence de SEQ ID NO : 13, 12A7 CDRL1 qui a une séquence de SEQ ID NO : 3, 12A7 CDRL2 qui a une séquence de SEQ ID NO : 5 et 12A7 CDRL3 qui a une séquence de SEQ ID NO : 7.

4. Anticorps de l'une quelconque des revendications 1 à 3, qui a une affinité pour Mcm5 dans la plage de 1 à 10 nM.

5. Anticorps de la revendication 1(ii) ou de la revendication 3, l'anticorps comprenant une région variable de chaîne lourde ayant une séquence identique à au moins 98 % à SEQ ID NO : 29, et l'anticorps comprenant une région variable de chaîne légère ayant une séquence identique à au moins 98 % à SEQ ID NO : 27.

6. Anticorps de la revendication 1(i) ou de la revendication 2, l'anticorps comprenant une région variable de chaîne lourde ayant une séquence identique à au moins 98 % à SEQ ID NO : 33, et l'anticorps comprenant une région variable de chaîne légère ayant une séquence identique à au moins 98 % à SEQ ID NO : 31.

7. Composition comprenant l'anticorps de l'une quelconque des revendications 1 à 6.

8. Composition comprenant :
(i) une séquence d'acides nucléiques identique à au moins 98 %, 99 % ou 100 % à SEQ ID NO : 28 et une séquence d'acides nucléiques identique à au moins 98 %, 99 % ou 100 % à SEQ ID NO : 30 ; ou
(ii) une séquence d'acides nucléiques identique à au moins 98 %, 99 % ou 100 % à SEQ ID NO : 32 et une séquence d'acides nucléiques identique à au moins 98 %, 99 % ou 100 % à SEQ ID NO : 34 ; ou
(iii) une séquence d'acides nucléiques de SEQ ID NO : 28 et une séquence d'acides nucléiques de SEQ ID NO : 30 ; ou
(iv) une séquence d'acides nucléiques de SEQ ID NO : 32 et une séquence d'acides nucléiques de SEQ ID NO : 34.

9. Cellule hôte comprenant l'acide nucléique de la revendication 8, la cellule hôte étant éventuellement une cellule de CHO ou une cellule de *E.coli.*

10. Procédé de fabrication d'un anticorps comprenant :
(i) la croissance d'une cellule hôte de la revendication 9 ; et
(ii) l'isolement d'un anticorps à partir de la cellule hôte.

11. Procédé de la revendication 10 :
(i) comprenant en outre une étape consistant à marquer l'anticorps par conjugaison de l'anticorps à un radiomarqueur ; ou
(ii) comprenant en outre une étape consistant à immobiliser l'anticorps sur un support solide.

12. Kit comprenant :
(i) une composition comprenant un premier anticorps selon l'une quelconque des revendications l(i), 2 ou 6 ; et
(ii) une composition comprenant un deuxième anticorps selon l'une quelconque des revendications l(ii), 3 ou 5.

13. Procédé de détermination de la concentration ou d'une quantité de Mcm5 dans un échantillon, comprenant les étapes suivantes :
(i) fournir un premier anticorps immobilisé qui est un anticorps de l'une quelconque des revendications 1 à 6 immobilisé sur un support solide ;
(ii) exposer l'échantillon au premier anticorps immobilisé de sorte que le Mcm5 dans l'échantillon puisse se lier à l'anticorps immobilisé pour fournir du Mcm5 immobilisé ;
(iii) fournir un deuxième anticorps marqué qui est un anticorps de l'une quelconque des revendications 1 à 6 conjugué à un marqueur ;
(iv) exposer le Mcm5 immobilisé au deuxième anticorps marqué de sorte que le deuxième anticorps marqué puisse se lier au Mcm5 ; et
(v) détecter la concentration du deuxième anticorps marqué ;
le premier anticorps et le deuxième anticorps se liant tous deux au Mcm5, mais le premier anticorps et le deuxième anticorps se liant à des épitopes différents du Mcm5.

14. Kit de la revendication 12 ou procédé de la revendication 13, dans lequel le premier anticorps et le deuxième anticorps se lient tous deux au Mcm5, mais le premier anticorps et le deuxième anticorps se lient à des épitopes différents du Mcm5 ; et
(a)
(i) le premier anticorps se lie à SEQ ID NO : 1 et le deuxième anticorps se lie à SEQ ID NO : 2 ; ou
(ii) le premier anticorps se lie à SEQ ID NO : 2 et le deuxième anticorps se lie à SEQ ID NO : 1 ; et/ou
(b) le premier anticorps est immobilisé sur une plaque ELISA ; et/ou
(c) le deuxième anticorps est immobilisé sur une plaque ELISA ; et/ou
(d) le deuxième anticorps est conjugué à un marqueur radioactif, le marqueur radioactif étant éventuellement l'Europium ³⁺ ; et/ou
(e) le premier anticorps est conjugué à un marqueur radioactif, le marqueur radioactif étant éventuellement l'Europium ³⁺.

15. Procédé de l'une quelconque des revendications 13 à 14, dans lequel l'étape consistant à détecter la concentration du deuxième anticorps marqué comprend la détection par immunofluorescence d'Eu³⁺.


### SEQUENCE LISTING

<110> Arquer Diagnostics Ltd
<120> MCM5 ASSAY
<130> N403141WO
<140> tbc
   <141> tbc
<150> tbc
   <151> tbc
<160> 36
<170> PatentIn version 3.5
<210> 1
   <211> 7
   <212> PRT
   <213> Artificial sequence
<220>
   <223> Epitope to which antibody 12A7 binds
<400> 1
   Trp Asp Glu Thr Lys Gly Glu
   1               5
<210> 2
   <211> 7
   <212> PRT
   <213> Artificial sequence
<220>
   <223> Epitope to which antibody 4B4 binds
<400> 2
   Asp Asp Arg Val Ala Ile His
   1               5
<210> 3
   <211> 11
   <212> PRT
   <213> Artificial sequence
<220>
   <223> 12A7 light chain CDR 1 polypeptide sequence
<400> 3
   Gln Asn Leu Val Gln Ser Asn Gly Asn Thr Tyr
   1               5                   10
<210> 4
   <211> 36
   <212> DNA
   <213> Artificial sequence
<220>
   <223> 12A7 light chain CDR 1 nucleotide sequence
<400> 4
   cagaaccttg tacaaagtaa tggaaacacc tattta 36
<210> 5
   <211> 4
   <212> PRT
   <213> Artificial sequence
<220>
   <223> 12A7 light chain CDR 2 polypeptide sequence
<220>
   <221> misc_feature
   <222> (4)..(4)
   <223> Xaa can be any naturally occurring amino acid
<400> 5
   Lys Val Ser Xaa
   1
<210> 6
   <211> 10
   <212> DNA
   <213> Artificial sequence
<220>
   <223> 12A7 light chain CDR 2 nucleotide sequence
<400> 6
   aagtttccaa 10
<210> 7
   <211> 9
   <212> PRT
   <213> Artificial sequence
<220>
   <223> 12A7 light chain CDR 3 polypeptide sequence
<400> 7
   Ser Gln Ser Thr Arg Val Pro Tyr Thr
   1 5
<210> 8
   <211> 27
   <212> DNA
   <213> Artificial sequence
<220>
   <223> 12A7 light chain CDR 3 nucleotide sequence
<400> 8
   tctcaaagta cacgtgttcc gtacaca 27
<210> 9
   <211> 10
   <212> PRT
   <213> Artificial sequence
<220>
   <223> 12A7 heavy chain CDR 1 polypeptide sequence
<400> 9
   Gly Phe Ser Leu Ser Thr Ser Gly Met Gly
   1 5 10
<210> 10
   <211> 30
   <212> DNA
   <213> Artificial sequence
<220>
   <223> 12A7 heavy chain CDR 1 nucleotide sequence
<400> 10
   gggttttcac tgagcacttc tggtatgggt 30
<210> 11
   <211> 7
   <212> PRT
   <213> Artificial sequence
<220>
   <223> 12A7 heavy chain CDR 2 polypeptide sequence
<400> 11
   Ile Phe Trp Asp Asp Asp Lys
   1 5
<210> 12
   <211> 21
   <212> DNA
   <213> Artificial sequence
<220>
   <223> 12A7 heavy chain CDR 2 nucleotide sequence
<400> 12
   attttctggg atgatgacaa g 21
<210> 13
   <211> 13
   <212> PRT
   <213> Artificial sequence
<220>
   <223> 12A7 heavy chain CDR 3 polypeptide sequence
<400> 13
   Ala Arg Arg Ser Asp Tyr Asn Tyr Tyr Ser Met Asp Tyr
   1 5 10
<210> 14
   <211> 39
   <212> DNA
   <213> Artificial sequence
<220>
   <223> 12A7 heavy chain CDR 3 nucleotide sequence
<400> 14
   gcgcggcgaa gtgactacaa ttactactct atggactac 39
<210> 15
   <211> 6
   <212> PRT
   <213> Artificial sequence
<220>
   <223> 4B4 light chain CDR 1 polypeptide sequence
<400> 15
   Gln Asp Ile Gly Ser Ser
   1 5
<210> 16
   <211> 18
   <212> DNA
   <213> Artificial sequence
<220>
   <223> 4B4 light chain CDR 1 nucleotide sequence
<400> 16
   caggacattg gtagtagc 18
<210> 17
   <211> 4
   <212> PRT
   <213> Artificial sequence
<220>
   <223> 4B4 light chain CDR 2 polypeptide sequence
<220>
   <221> misc_feature
   <222> (4)..(4)
   <223> Xaa can be any naturally occurring amino acid
<400> 17
   Ala Thr Ser Xaa
   1
<210> 18
   <211> 10
   <212> DNA
   <213> Artificial sequence
<220>
   <223> 4B4 light chain CDR 2 nucleotide sequence
<220>
   <221> misc_feature
   <222> (10)..(10)
   <223> n is a, c, g, or t
<400> 18
   gccacatccn 10
<210> 19
   <211> 9
   <212> PRT
   <213> Artificial sequence
<220>
   <223> 4B4 light chain CDR 3 polypeptide sequence
<400> 19
   Leu Gln Tyr Ala Ser Ser Pro Pro Thr
   1               5
<210> 20
   <211> 27
   <212> DNA
   <213> Artificial sequence
<220>
   <223> 4B4 light chain CDR 3 nucleotide sequence
<400> 20
   ctacaatatg ctagttctcc tccgacg 27
<210> 21
   <211> 8
   <212> PRT
   <213> Artificial sequence
<220>
   <223> 4B4 heavy chain CDR 1 polypeptide sequence
<400> 21
   Gly Phe Thr Phe Ser Asn Tyr Ala
   1               5
<210> 22
   <211> 24
   <212> DNA
   <213> Artificial sequence
<220>
   <223> 4B4 heavy chain CDR 1 nucleotide sequence
<400> 22
   ggattcactt tcagtaacta tgcc 24
<210> 23
   <211> 8
   <212> PRT
   <213> Artificial sequence
<220>
   <223> 4B4 heavy chain CDR 2 polypeptide sequence
<400> 23
   Ile Ser Arg Gly Gly Ser Tyr Thr
   1               5
<210> 24
   <211> 24
   <212> DNA
   <213> Artificial sequence
<220>
   <223> 4B4 heavy chain CDR 2 nucleotide sequence
<400> 24
   attagtcgtg gtggtagtta cacc 24
<210> 25
   <211> 15
   <212> PRT
   <213> Artificial sequence
<220>
   <223> 4B4 heavy chain CDR 3 polypeptide sequence
<400> 25
   Ala Arg His Gly Tyr Asn Tyr Asp Asp Gly Ala Trp Phe Ala Asn
   1               5                   10                  15
<210> 26
   <211> 45
   <212> DNA
   <213> Artificial sequence
<220>
   <223> 4B4 heavy chain CDR 3 nucleotide sequence
<400> 26
   gcaagacatg gatataatta cgacgacggg gcctggtttg ctaac 45
<210> 27
   <211> 113
   <212> PRT
   <213> Artificial sequence
<220>
   <223> 12A7 full variable light chain sequence (polypeptide)
<400> 27
   Asp Ile Met Leu Thr Gln Ser Pro Leu Ser Leu Ser Val Thr Leu Gly
   1               5                   10                  15
   Asp Gln Ala Ser Ile Ser Cys Arg Ser Ser Gln Asn Leu Val Gln Ser
               20                  25                  30
   Asn Gly Asn Thr Tyr Leu Thr Trp Tyr Leu Gln Lys Pro Gly Gln Ser
           35                  40                  45
   Pro Lys Val Leu Ile Asn Lys Val Ser Asn Arg Phe Tyr Gly Val Pro
       50                  55                  60
   Asp Arg Phe Ser Gly Ser Gly Ser Gly Thr Asp Phe Thr Leu Arg Ile
   65                  70                  75                  80
   Ser Arg Val Glu Ala Glu Asp Leu Gly Ile Tyr Phe Cys Ser Gln Ser
                   85                  90                  95
   Thr Arg Val Pro Tyr Thr Phe Gly Gly Gly Thr Lys Leu Glu Ile Arg
               100                 105                 110
   Arg
<210> 28
   <211> 338
   <212> DNA
   <213> Artificial sequence
<220>
   <223> 12A7 full variable light chain sequence (nucleotide)
<400> 28
   gatatcatgc tgacccaatc tccactctcc ctgtctgtca ctcttggaga tcaggcctcc 60
   atctcttgca gatctagtca gaaccttgta caaagtaatg gaaacaccta tttaacttgg 120
   tacctgcaga agccaggcca gtctccaaag gtcctgatca acaaagtttc caaccgattt 180
   tatggggtcc cagacaggtt cagtggcagt ggatcaggga cagatttcac actcaggatc 240
   agcagagtgg aggctgagga tctgggaatt tatttctgct ctcaaagtac acgtgttccg 300
   tacacattcg gaggggggac caagctggaa ataagacg 338
<210> 29
   <211> 121
   <212> PRT
   <213> Artificial sequence
<220>
   <223> 12A7 full variable heavy chain sequence (polypeptide)
<400> 29
   Gln Gln Asp Leu Gln Gln Ser Gly Pro Gly Ile Leu Gln Pro Thr Gln
   1               5                   10                  15
   Thr Leu Ser Leu Thr Cys Ser Phe Ser Gly Phe Ser Leu Ser Thr Ser
               20                  25                  30
   Gly Met Gly Val Ser Trp Ile Arg Gln Ser Ser Asn Met Gly Leu Glu
           35                  40                  45
   Trp Leu Ala His Ile Phe Trp Asp Asp Asp Lys Arg Tyr Asn Pro Ser
       50                  55                  60
   Leu Arg Ser Arg Leu Thr Leu Ser Lys Asp Thr Ser Ser Ser Gln Val
   65                  70                  75                  80
   Phe Leu Met Ile Thr Ser Val Ser Thr Ala Asp Ser Ala Thr Tyr Tyr
                   85                  90                  95
   Cys Ala Arg Arg Ser Asp Tyr Asn Tyr Tyr Ser Met Asp Tyr Trp Gly
               100                 105                 110
   Gln Gly Thr Ala Val Thr Val Ser Ser
           115                 120
<210> 30
   <211> 363
   <212> DNA
   <213> Artificial sequence
<220>
   <223> 12A7 full variable heavy chain sequence (nucleotide)
<400> 30
   cagcaagatc tgcagcagtc tggccctggg atattgcagc ccacccagac cctcagtctg      60
   acttgttctt tctctgggtt ttcactgagc acttctggta tgggtgtgag ttggattcgt     120
   caatcttcaa atatgggtct ggagtggctg gcacacattt tctgggatga tgacaagcgc     180
   tataatccct ccctgaggag ccgactcacg ctctccaagg atacctccag tagccaggta     240
   tttctcatga tcaccagtgt gagtactgca gattctgcca catactactg tgcgcggcga     300
   agtgactaca attactactc tatggactac tggggtcaag gaaccgcagt caccgtctcc     360
   tca                                                                   363
<210> 31
   <211> 107
   <212> PRT
   <213> Artificial sequence
<220>
   <223> 4B4 full variable light chain sequence (polypeptide)
<400> 31
   Asp Ile Met Leu Thr Gln Ser Pro Ser Ser Leu Ser Ala Ser Leu Gly
   1               5                   10                  15
   Glu Arg Val Ser Leu Thr Cys Arg Ala Ser Gln Asp Ile Gly Ser Ser
               20                  25                  30
   Leu Asn Trp Leu Gln Gln Glu Pro Asp Gly Thr Ile Lys Arg Leu Ile
           35                  40                  45
   Tyr Ala Thr Ser Ser Leu Asp Ser Gly Val Pro Lys Arg Phe Ser Gly
       50                  55                  60
   Ser Arg Ser Gly Ser Asp Tyr Ser Leu Thr Ile Ser Ser Leu Glu Ser
   65                  70                  75                  80
   Glu Asp Phe Val Asp Tyr Tyr Cys Leu Gln Tyr Ala Ser Ser Pro Pro
                   85                  90                  95
   Thr Phe Gly Gly Gly Thr Lys Leu Glu Ile Lys
               100                 105
<210> 32
   <211> 322
   <212> DNA
   <213> Artificial sequence
<220>
   <223> 4B4 full variable light chain sequence (nucleotide)
<400> 32
   gatatcatgc tgacccaatc tccatcctcc ttatctgcct ctctgggaga aagagtcagt        60
   ctcacttgtc gggcaagtca ggacattggt agtagcttaa actggcttca acaggaacca       120
   gatggaacta ttaaacgcct aatctacgcc acatccagtt tagattctgg tgtccccaaa       180
   aggttcagtg gcagtaggtc tgggtcagat tattctctca ccatcagcag ccttgagtct       240
   gaagattttg tagactatta ctgtctacaa tatgctagtt ctcctccgac gttcggtgga       300
   ggcaccaagc tggaaatcaa ac                                                322
<210> 33
   <211> 121
   <212> PRT
   <213> Artificial sequence
<220>
   <223> 4B4 full variable heavy chain sequence (polypeptide)
<400> 33
   Val Lys Leu Gln Glu Ser Gly Gly Gly Leu Val Lys Pro Gly Gly Ser
   1               5                   10                  15
   Leu Lys Leu Ser Cys Ala Ala Ser Gly Phe Thr Phe Ser Asn Tyr Ala
               20                  25                  30
   Met Ser Trp Val Arg Gln Asn Pro Glu Lys Arg Leu Glu Trp Val Ala
           35                  40                  45
   Thr Ile Ser Arg Gly Gly Ser Tyr Thr Tyr Tyr Pro Asp Ser Val Lys
       50                  55                  60
   Gly Arg Phe Thr Ile Ser Arg Asp Asn Ala Lys Asn Thr Leu Tyr Leu
   65                  70                  75                  80
   Gln Met Asn Ser Leu Arg Ser Glu Asp Thr Ala Met Tyr Phe Cys Ala
                   85                  90                  95
   Arg His Gly Tyr Asn Tyr Asp Asp Gly Ala Trp Phe Ala Asn Trp Gly
               100                 105                 110
   Gln Gly Thr Leu Val Thr Val Ser Ala
           115                 120
<210> 34
   <211> 366
   <212> DNA
   <213> Artificial sequence
<220>
   <223> 4B4 full variable heavy chain sequence (nucleotide)
<400> 34
   taggtgaaac tgcaggagtc tgggggaggc ttagtgaagc ctggagggtc cctgaaactc        60
   tcctgtgcag cctctggatt cactttcagt aactatgcca tgtcttgggt tcgccagaat       120
   ccggagaaga ggctggagtg ggtcgcaacc attagtcgtg gtggtagtta cacctactat       180
   ccagacagtg tgaagggtcg attcaccatc tccagagaca atgccaagaa caccctgtat       240
   ctgcaaatga acagtctgag gtctgaggac acggccatgt atttctgtgc aagacatgga       300
   tataattacg acgacggggc ctggtttgct aactggggcc aagggactct ggtcactgtc       360
   tctgca                                                                  366
<210> 35
   <211> 734
   <212> PRT
   <213> Homo sapiens
<220>
   <223> Mcm5 (polypeptide)
<400> 35
   Met Ser Gly Phe Asp Asp Pro Gly Ile Phe Tyr Ser Asp Ser Phe Gly
   1               5                   10                  15
   Gly Asp Ala Gln Ala Asp Glu Gly Gln Ala Arg Lys Ser Gln Leu Gln
               20                  25                  30
   Arg Arg Phe Lys Glu Phe Leu Arg Gln Tyr Arg Val Gly Thr Asp Arg
           35                  40                  45
   Thr Gly Phe Thr Phe Lys Tyr Arg Asp Glu Leu Lys Arg His Tyr Asn
       50                  55                  60
   Leu Gly Glu Tyr Trp Ile Glu Val Glu Met Glu Asp Leu Ala Ser Phe
   65                  70                  75                  80
   Asp Glu Asp Leu Ala Asp Tyr Leu Tyr Lys Gln Pro Ala Glu His Leu
                   85                  90                  95
   Gln Leu Leu Glu Glu Ala Ala Lys Glu Val Ala Asp Glu Val Thr Arg
               100                 105                 110
   Pro Arg Pro Ser Gly Glu Glu Val Leu Gln Asp Ile Gln Val Met Leu
           115                 120                 125
   Lys Ser Asp Ala Ser Pro Ser Ser Ile Arg Ser Leu Lys Ser Asp Met
       130                 135                 140
   Met Ser His Leu Val Lys Ile Pro Gly Ile Ile Ile Ala Ala Ser Ala
   145                 150                 155                 160
   Val Arg Ala Lys Ala Thr Arg Ile Ser Ile Gln Cys Arg Ser Cys Arg
                   165                 170                 175
   Asn Thr Leu Thr Asn Ile Ala Met Arg Pro Gly Leu Glu Gly Tyr Ala
               180                 185                 190
   Leu Pro Arg Lys Cys Asn Thr Asp Gln Ala Gly Arg Pro Lys Cys Pro
           195                 200                 205
   Leu Asp Pro Tyr Phe Ile Met Pro Asp Lys Cys Lys Cys Val Asp Phe
       210                 215                 220
   Gln Thr Leu Lys Leu Gln Glu Leu Pro Asp Ala Val Pro His Gly Glu
   225                 230                 235                 240
   Met Pro Arg His Met Gln Leu Tyr Cys Asp Arg Tyr Leu Cys Asp Lys
                   245                 250                 255
   Val Val Pro Gly Asn Arg Val Thr Ile Met Gly Ile Tyr Ser Ile Lys
               260                 265                 270
   Lys Phe Gly Leu Thr Thr Ser Arg Gly Arg Asp Arg Val Gly Val Gly
           275                 280                 285
   Ile Arg Ser Ser Tyr Ile Arg Val Leu Gly Ile Gln Val Asp Thr Asp
       290                 295                 300
   Gly Ser Gly Arg Ser Phe Ala Gly Ala Val Ser Pro Gln Glu Glu Glu
   305                 310                 315                 320
   Glu Phe Arg Arg Leu Ala Ala Leu Pro Asn Val Tyr Glu Val Ile Ser
                   325                 330                 335
   Lys Ser Ile Ala Pro Ser Ile Phe Gly Gly Thr Asp Met Lys Lys Ala
               340                 345                 350
   Ile Ala Cys Leu Leu Phe Gly Gly Ser Arg Lys Arg Leu Pro Asp Gly
           355                 360                 365
   Leu Thr Arg Arg Gly Asp Ile Asn Leu Leu Met Leu Gly Asp Pro Gly
       370                 375                 380
   Thr Ala Lys Ser Gln Leu Leu Lys Phe Val Glu Lys Cys Ser Pro Ile
   385                 390                 395                 400
   Gly Val Tyr Thr Ser Gly Lys Gly Ser Ser Ala Ala Gly Leu Thr Ala
                   405                 410                 415
   Ser Val Met Arg Asp Pro Ser Ser Arg Asn Phe Ile Met Glu Gly Gly
               420                 425                 430
   Ala Met Val Leu Ala Asp Gly Gly Val Val Cys Ile Asp Glu Phe Asp
           435                 440                 445
   Lys Met Arg Glu Asp Asp Arg Val Ala Ile His Glu Ala Met Glu Gln
       450                 455                 460
   Gln Thr Ile Ser Ile Ala Lys Ala Gly Ile Thr Thr Thr Leu Asn Ser
   465 470 475 480
   Arg Cys Ser Val Leu Ala Ala Ala Asn Ser Val Phe Gly Arg Trp Asp
   485 490 495
   Glu Thr Lys Gly Glu Asp Asn Ile Asp Phe Met Pro Thr Ile Leu Ser
   500 505 510
   Arg Phe Asp Met Ile Phe Ile Val Lys Asp Glu His Asn Glu Glu Arg
   515 520 525
   Asp Val Met Leu Ala Lys His Val Ile Thr Leu His Val Ser Ala Leu
   530 535 540
   Thr Gln Thr Gln Ala Val Glu Gly Glu Ile Asp Leu Ala Lys Leu Lys
   545 550 555 560
   Lys Phe Ile Ala Tyr Cys Arg Val Lys Cys Gly Pro Arg Leu Ser Ala
   565 570 575
   Glu Ala Ala Glu Lys Leu Lys Asn Arg Tyr Ile Ile Met Arg Ser Gly
   580 585 590
   Ala Arg Gln His Glu Arg Asp Ser Asp Arg Arg Ser Ser Ile Pro Ile
   595 600 605
   Thr Val Arg Gln Leu Glu Ala Ile Val Arg Ile Ala Glu Ala Leu Ser
   610 615 620
   Lys Met Lys Leu Gln Pro Phe Ala Thr Glu Ala Asp Val Glu Glu Ala
   625 630 635 640
   Leu Arg Leu Phe Gln Val Ser Thr Leu Asp Ala Ala Leu Ser Gly Thr
   645 650 655
   Leu Ser Gly Val Glu Gly Phe Thr Ser Gln Glu Asp Gln Glu Met Leu
   660 665 670
   Ser Arg Ile Glu Lys Gln Leu Lys Arg Arg Phe Ala Ile Gly Ser Gln
   675 680 685
   Val Ser Glu His Ser Ile Ile Lys Asp Phe Thr Lys Gln Lys Tyr Pro
   690 695 700
   Glu His Ala Ile His Lys Val Leu Gln Leu Met Leu Arg Arg Gly Glu
   705 710 715 720
   Ile Gln His Arg Met Gln Arg Lys Val Leu Tyr Arg Leu Lys
   725 730
<210> 36
   <211> 2534
   <212> DNA
   <213> Homo sapiens
<220>
   <223> Mcm5 (mRNA)
<400> 36
   ggaaaaccag aggcgcagtc atgtcgggat tcgacgatcc tggcattttc tacagcgaca      60
   gcttcggggg cgacgcccag gccgacgagg ggcaggcccg caaatcgcag ctgcagaggc     120
   gcttcaagga gttcctgcgg cggtaccgag tgggcaccga ccgcacgggc ttcaccttca     180
   aatacaggga tgaactcaag cggcattaca acctggggga gtactggatt gaggtggaga     240
   tggaggatct ggccagcttt gatgaggacc tggccgacta cttgtacaag cagccagccg     300
   agcacctgca gctgctggag gaagctgcca aggaggtagc tgatgaggtg acccggcccc     360
   ggccttctgg ggaggaggtg ctccaggaca tccaggtcat gctcaagtcg gacgccagcc     420
   cttccagcat tcgtagcctg aagtcggaca tgatgtcaca cctggtgaag atccctggca     480
   tcatcatcgc ggcctctgcg gtccgtgcca aggccacccg catctctatc cagtgccgca     540
   gctgccgcaa caccctcacc aacattgcca tgcgccctgg cctcgagggc tatgccctgc     600
   ccaggaagtg caacacagat caggctgggc gccccaaatg cccattggac ccgtacttca     660
   tcatgcccga caaatgcaaa tgcgtggact tccagaccct gaagctgcag gagctgcctg     720
   atgcagtccc ccacggggag atgcccagac acatgcagct ctactgcgac aggtacctgt     780
   gtgacaaggt cgtccctggg aacagggtta ccatcatggg catctactcc atcaagaagt     840
   ttggcctgac taccagcagg ggccgtgaca gggtgggcgt gggcatccga agctcctaca     900
   tccgtgtcct gggcatccag gtggacacag atggctctgg ccgcagcttt gctggggccg     960
   tgagccccca ggaggaggag gagttccgtc gcctggctgc cctcccaaat gtctatgagg    1020
   tcatctccaa gagcatcgcc ccctccatct ttgggggcac agacatgaag aaggccattg    1080
   cctgcctgct ctttgggggc tcccgaaaga ggctccctga tggacttact cgccgaggag    1140
   acatcaacct gctgatgcta ggggaccctg ggacagccaa gtcccagctt ctgaagtttg    1200
   tggagaagtg ttctcccatt ggggtataca cgtctgggaa aggcagcagc gcagctggac    1260
   tgacagcctc ggtgatgagg gacccttcgt cccggaattt catcatggag ggcggagcca    1320
   tggtcctggc cgatggtggg gtcgtctgta ttgacgagtt tgacaagatg cgagaagatg    1380
   accgtgtggc aatccacgaa gccatggagc agcagaccat ctctatcgcc aaggctggga    1440
   tcaccaccac cctgaactcc cgctgctccg tcctggctgc tgccaactca gtgttcggcc     1500
   gctgggatga gacgaagggg gaggacaaca ttgacttcat gcccaccatc ttgtcgcgct     1560
   tcgacatgat cttcatcgtc aaggatgagc acaatgagga gagggatgtg atgctggcca     1620
   agcatgtcat cactctgcac gtgagcgcac tgacacagac acaggctgtg gagggcgaga     1680
   ttgacctggc caagctgaag aagtttattg cctactgccg agtgaagtgt ggcccccggc     1740
   tgtcagcaga ggctgcagag aaactgaaga accgctacat catcatgcgg agcggggccc     1800
   gtcagcacga gagggacagt gaccgccgct ccagcatccc catcactgtg cggcagctgg     1860
   aggccattgt gcgcatcgcg gaagccctca gcaagatgaa gctgcagccc ttcgccacag     1920
   aggcagatgt ggaggaggcc ctgcggctct tccaagtgtc cacgttggat gctgccttgt     1980
   ccggtaccct gtcaggggtg gagggcttca ccagccagga ggaccaggag atgctgagcc     2040
   gcatcgagaa gcagctcaag cgccgctttg ccattggctc ccaggtgtct gagcacagca     2100
   tcatcaagga cttcaccaag cagaaatacc cggagcacgc catccacaag gtgctgcagc     2160
   tcatgctgcg gcgcggcgag atccagcatc gcatgcagcg caaggttctc taccgcctca     2220
   agtgagtcgc gccgcctcac tggactcatg gactcgccca cgcctcgccc ctcctgccgc     2280
   tgcctgccat tgacaatgtt gctgggacct ctgcctcccc actgcagccc tcgaacttcc     2340
   caggcaccct cctttctgcc ccagaggaag gagctgtagt gtcctgctgc ctctgggcgc     2400
   ccgcctctag cgcggttctg ggaagtgtgc ttttggcatc cgttaataat aaagccacgg     2460
   tgtgttcagg taaaaaaaaa aaaaaaaaaa aaaaaaaaaa aaaaaaaaaa aaaaaaaaaa     2520
   aaaaaaaaaa aaaa                                                       2534